# EUROPEAN PATENT SPECIFICATION

(11) **EP 4 165 648 B1**
(45) Date of publication and mention of the grant of the patent: **02.12.2026**
(21) Application number: 21736943.8
(22) Date of filing: 10.06.2021
(51) Int. Cl.: G16H 20/17, G16H 50/20, A61B 5/145

(54) **DETECTING MEAL INGESTION OR MISSED BOLUS**
ERKENNUNG VON NAHRUNGSAUFNAHME ODER VERPASSTEM BOLUS
DÉTECTION D'INGESTION DE REPAS OU DE BOLUS MANQUÉ

(30) Priority: 10.06.2020 US 202062705089 P
(43) Date of publication of application: 19.04.2023
(73) Proprietor: Bigfoot Biomedical, Inc., Milpitas, CA 95035 (US)
(72) Inventor: BENNETT, Paul Curtis, Milpitas, California 95035 (US)
(74) Representative: Murgitroyd & Company
(86) International application number: PCT/US2021/070690
(87) International publication number: WO 2021/253046

(56) References cited:
- WO-A1-2019/157102
- WO-A1-2020/252496
- WO-A1-2020/263706
- US-A1- 2010 094 251
- US-A1- 2019 307 957

## Description

### CROSS REFERENCE TO RELATED APPLICATION

This application claims priority to U.S. Provisional Application No. 62/705,089, filed on June 10, 2020, entitled "DETECTING MEAL INGESTION OR MISSED BOLUS,".

### TECHNICAL FIELD

This document relates to detecting meal ingestion and/or a missed bolus.

### BACKGROUND

Diabetes mellitus is a chronic metabolic disorder caused by the inability of a person's pancreas to produce sufficient amounts of the hormone insulin such that the person's metabolism is unable to provide for the proper absorption of sugar and starch. This failure leads to hyperglycemia, i.e., the presence of an excessive amount of glucose within the blood plasma. Persistent hyperglycemia has been associated with a variety of serious symptoms and life threatening long-term complications such as dehydration, ketoacidosis, diabetic coma, cardiovascular diseases, chronic renal failure, retinal damage and nerve damages with the risk of amputation of extremities. Because healing is not yet possible, a permanent therapy is necessary which provides constant glycemic control in order to constantly maintain the level of blood analyte within normal limits. Such glycemic control is achieved by regularly supplying external drugs to the body of the patient to thereby reduce the elevated levels of blood analyte. An external biologically effective drug (e.g., insulin or its analog) is commonly administered by means of daily injections. In some cases, multiple, daily injections of a mixture of rapid- and long-acting insulin are administered via a reusable transdermal liquid dosing device.

To determine a missed bolus, prior art US2010/094251A1 evaluates a local minimum on a glucose curve or change in slope of the glucose curve.

### SUMMARY

An aspect of the invention defined in claim 1, and optional dependent claims 2 to 14 comprises a computer-implemented method of detecting a missed bolus. The computer-implemented method may comprise: receiving blood glucose values and bolus information regarding a person with diabetes, the blood glucose values and the bolus information relating to a period of time; classifying, based on the blood glucose values and the bolus information, each of multiple time periods within the period of time regarding whether the time period is associated with ingestion of a meal by the person; performing regression analysis on the classified multiple time periods to identify a first time period of the classified multiple time periods as corresponding to a beginning of the meal, wherein the bolus information indicates no bolus associated with the identified first time period; and associating, based on the regression analysis, a missed bolus event with the meal.

Implementations can include any or all of the following features. The computer-implemented method further comprises performing at least one action based on the missed bolus event being associated with the meal. The action includes issuing an alert regarding the person. The blood glucose values and the bolus information are received substantially in real time, and wherein the alert is issued substantially in real time with the bolus being missed. Issuing the alert in real time comprises waiting a predetermined time after the beginning of the meal before determining that the bolus was missed. Classifying the multiple time periods comprises providing the blood glucose values and the bolus information to a first machine-learning set including at least one classifier, and wherein performing the regression analysis comprises providing at least an output of the first machine-learning set to a second machine-learning set including at least one regressor. The first machine-learning set includes multiple classifiers receiving also the blood glucose values and the bolus information, and wherein classifying each of the multiple time periods comprises generating one classification event for each of the multiple classifiers for each of the multiple time periods. The computer-implemented method further comprises providing each of the multiple classifiers with an aggregation of at least one of the blood glucose values or the bolus information. Receiving the bolus information comprises receiving data generated by a pen cap based on the pen cap detecting at least one of a removal of the pen cap from an insulin pen, or a replacement of the pen cap on the insulin pen. Receiving the bolus information comprises receiving amount information corresponding to an amount of insulin administered to the person.

The computer-implemented method further comprises: generating, based on the blood glucose values and the bolus information, a meal probability density for the person; determining, based on the bolus information and the meal probability density, an expected bolus amount; comparing the amount information with the expected bolus amount; and issuing an alert regarding the person based on a discrepancy between the amount information and the expected bolus amount. The computer-implemented method further comprises generating, based on the blood glucose values and the bolus information, a meal probability density for the person. The computer-implemented method further comprises generating multiple meal probability densities corresponding to different days of a week for the person. The meal probability density reflects a likelihood of meal ingestion with regard to time. The computer-implemented method further comprises taking into account the meal probability density before associating the missed bolus event with the meal. The computer-implemented method further comprises issuing an alert regarding the person based on the missed bolus event being associated with the meal. The blood glucose values and the bolus information are received substantially in real time, and wherein the alert is issued substantially in real time with the bolus being missed. The computer-implemented method further comprises taking into account the meal probability density before issuing the alert, wherein a relatively lower threshold for the alert is used when a current meal probability is relatively higher, wherein a relatively higher threshold for the alert is used when a current meal probability is relatively lower. Taking into account the meal probability density comprises changing a threshold for a feature in a machine-learning classifier based on a kernel smoothed density estimate in the meal probability density. Taking into account the meal probability density comprises reducing a threshold for associating the missed bolus event with the meal. Generating the meal probability density comprises using a bolus density as a proxy for meal occurrence likelihood. The computer-implemented method further comprises generating the bolus density by aggregating bolus events for the person that are included in the bolus information. The bolus events are distributed within the period of time, and wherein aggregating the bolus events comprises: wrapping the bolus events over a time interval shorter than the period of time so that the bolus events are distributed within the time interval; generating, based on the bolus events wrapped over the time interval, a continuous distribution of likelihood over the time interval; and replicating the continuous distribution of likelihood at least once within the period of time. Generating the continuous distribution of likelihood comprises smoothing the bolus events wrapped over the time interval. Smoothing the bolus events comprises performing a kernel smoothed density estimate. Generating the continuous distribution of likelihood comprises filtering the bolus events wrapped over the time interval.

The computer-implemented method further comprises generating, based on the blood glucose values and the bolus information, a bolus density for the person. Generating the bolus density comprises using a meal probability density for the person. The bolus information includes bolus events that are delta functions, the method further comprising broadening, before classifying the multiple time periods, each of the bolus events to have a finite time duration. Broadening each of the bolus events comprises convolving the delta function with a rectangle function. No bolus being associated with the identified first time period comprises that the bolus information includes no bolus separated from the identified first time period by at most a predefined time, and wherein the finite time duration is a multiple of the predefined time. The computer-implemented method further comprises aggregating, before classifying the multiple time periods, the bolus events in multiple overlapping time intervals. The blood glucose values and the bolus information are received substantially in real time. The blood glucose values and the bolus information are received in batch. No bolus being associated with the identified first time period comprises that the bolus information includes no bolus separated from the identified first time period by at most a predefined time, and wherein the bolus information does include a bolus separated from the identified first time period by more than the predefined time, the method further comprising associating a late bolus event with the bolus.

An aspect of the invention defined in claim 15 relates to an insulin management system. The insulin management system may comprise: an input device to receive blood glucose values and bolus information regarding a person with diabetes, the blood glucose values and the bolus information relating to a period of time; a classifier implemented by at least one processor, the classifier to classify, based on the blood glucose values and the bolus information, each of multiple time periods within the period of time regarding whether the time period is associated with ingestion of a meal by the person; and a regressor implemented by at least one processor, the regressor to perform regression analysis on the classified multiple time periods to identify a first time period of the classified multiple time periods as corresponding to a beginning of the meal, wherein the bolus information indicates no bolus associated with the identified first time period; wherein the insulin treatment device associates, based on the regression analysis, a missed bolus event with the meal.

Implementations can include any or all of the following features. The insulin treatment device comprises at least one of a smartphone, a pen cap for an insulin pen, an insulin pen, an insulin pump, a continuous glucose monitor, or a blood glucose meter. The insulin treatment device further comprises a user interface, wherein the insulin treatment device is configured for issuing an alert regarding the person using the user interface, the alert issued based on the missed bolus event being associated with the meal.

An aspect of the invention defined in claim 16 relates to a computer program product stored in a non-transitory storage medium, the computer program product including instructions that when executed by at least one processor cause the at least one processor to perform operations. The instructions may cause the at least one processor to perform operations comprising: receiving blood glucose values and bolus information regarding a person with diabetes, the blood glucose values and the bolus information relating to a period of time; classifying, based on the blood glucose values and the bolus information, each of multiple time periods within the period of time regarding whether the time period is associated with ingestion of a meal by the person; performing regression analysis on the classified multiple time periods to identify a first time period of the classified multiple time periods as corresponding to a beginning of the meal, wherein the bolus information indicates no bolus associated with the identified first time period; and associating, based on the regression analysis, a missed bolus event with the meal.

In an aspect not defined in the claims, a computer-implemented method of determining a personalized meal propensity comprises: receiving bolus information regarding a person with diabetes, the bolus information comprising bolus events distributed within a period of time; wrapping the bolus information over a time interval shorter than the period of time so that the bolus events are distributed within the time interval; and generating a meal probability density based on the wrapped bolus information, wherein the meal probability density is a continuous function of time over the time interval.

Implementations can include any or all of the following features. Generating the meal probability density comprises taking into account a missed bolus during the period of time. The computer-implemented method further comprises determining the missed bolus before taking into account the missed bolus in generating the meal probability density. Determining the missed bolus comprises: receiving blood glucose values regarding the person with diabetes, the blood glucose values relating to the period of time; classifying, based on the blood glucose values and the bolus information, each of multiple time periods within the period of time regarding whether the time period is associated with ingestion of a meal by the person; performing regression analysis on the classified multiple time periods to identify a first time period of the classified multiple time periods as corresponding to a beginning of the meal, wherein the bolus information indicates no bolus associated with the identified first time period; and associating, based on the regression analysis, a missed bolus event with the meal. The computer-implemented method further comprises performing at least one action based on the meal probability density. The action includes issuing an alert regarding the person. The meal probability density is generated by at least one processor implemented in a pen cap of an insulin pen, and wherein receiving the bolus information comprises receiving data generated by the pen cap based on the pen cap detecting at least one of a removal of the pen cap from the insulin pen, or a replacement of the pen cap on the insulin pen. The bolus information includes bolus events that are delta functions, the method further comprising broadening, before generating the meal probability density, each of the bolus events to have a finite time duration. Broadening each of the bolus events comprises convolving the delta function with a rectangle function. The computer-implemented method further comprises aggregating, before generating the meal probability density, the bolus events in multiple overlapping time intervals. The continuous function extends over the time interval, the method further comprising replicating the continuous function over multiple instances of the time interval so that the continuous function extends throughout the period of time.

In an aspect not defined in the claims, an insulin treatment device comprises: at least one processor; and a memory including instructions that when executed by the at least one processor cause the at least one processor to perform operations comprising: receiving bolus information regarding a person with diabetes, the bolus information comprising bolus events distributed within a period of time; wrapping the bolus information over a time interval shorter than the period of time so that the bolus events are distributed within the time interval; and generating a meal probability density based on the wrapped bolus information, wherein the meal probability density is a continuous function of time over the time interval.

Implementations can include any or all of the following features. The insulin treatment device comprises at least one of a smartphone, a pen cap for an insulin pen, an insulin pen, an insulin pump, a continuous glucose monitor, or a blood glucose meter. The insulin treatment device further comprises a user interface, wherein the operations further include issuing an alert regarding the person using the user interface, the alert issued based on the meal probability density.

In an aspect not defined in the claims, a computer program product is stored in a non-transitory storage medium, the computer program product including instructions that when executed by at least one processor cause the at least one processor to perform operations comprising: receiving bolus information regarding a person with diabetes, the bolus information comprising bolus events distributed within a period of time; wrapping the bolus information over a time interval shorter than the period of time so that the bolus events are distributed within the time interval; and generating a meal probability density based on the wrapped bolus information, wherein the meal probability density is a continuous function of time over the time interval.

### BRIEF DESCRIPTION OF DRAWINGS

FIG. 1 shows an example of a system that can detect a meal and/or a missed bolus.
FIG. 2 shows an example of input and output for meal and/or missed-bolus detection.
FIG. 3 shows an example of feature generation, and aggregation and scaling of one or more features.
FIG. 4 shows an example of features relating to estimated glucose value and rates of change.
FIG. 5 shows an example of spreading out data of a feature.
FIG. 6A schematically shows an example of aggregating bolus information.
FIG. 6B shows an example of a meal probability density.
FIG. 7 schematically shows an example of aggregating feature information into aggregated feature information.
FIG. 8 schematically shows an example of classification based on a feature.
FIG. 9 schematically shows an example of regression based on classification.
FIG. 10 shows an example of training data reflecting distribution of a blood protein within a population.
FIG. 11 shows an example of a system for diabetes management.
FIG. 12 is a perspective view of an example of an infusion pump system.
FIGS. 13A-B show examples of graphical user interfaces.
FIG. 14 shows a simplified block diagram of an example of a computing platform for insulin-based management of diabetes.
FIG. 15 shows a simplified block diagram of an example of a system for insulin-based management of diabetes.
FIG. 16 shows a diagram of an example of a system for diabetes management.
FIG. 17 shows an example of a system for training a regression model.
FIG. 18 shows a functional block diagram of an example of a system for training a regression model using machine learning.
FIG. 19 illustrates an example of a data journey in accordance with one embodiment.
FIG. 20 illustrates an example architecture of a computing device that can be used to implement aspects of the present disclosure.

Like reference symbols in the various drawings indicate like elements.

### DETAILED DESCRIPTION

This document describes examples of systems and techniques for detecting meal ingestion and/or a missed bolus with regard to a person with diabetes. In some implementations, one or more approaches for machine-learning can be applied to determine, for a period of time, a bolus density for the person with diabetes. For example, a meal probability density for the person can be generated based on the bolus density. The detection of the meal ingestion and/or the missed bolus can be used for one or more purposes. In some implementations, a diabetes treatment can be implemented or altered based on the detection. For example, an alert, recommendation, and/or bolus recommendation can be made based on the detection.

Examples described herein mention delivering insulin to a person with diabetes. Insulin delivery devices include, but are not limited to, insulin injection pens, insulin inhalers, insulin pumps, and insulin syringes. The improper dosing of insulin, whether due to human error, malfunction of an insulin pen, skipping doses, double dosing, or incorrect dosing, is always a concern. Methods, devices, and systems provided herein are described for the delivery of insulin, collection of blood glucose data, and/or the treatment of diabetes. Moreover, methods, devices, and systems provided herein may be adapted for the delivery of other medications, the collection of other analyte data, and/or the treatment of other diseases. Methods, devices, and systems provided herein are described by exemplifying features and functionalities of a number of illustrative embodiments. Other implementations are also possible.

Some examples herein refer to an insulin injection pen. An insulin injection pen includes at least one container holding insulin (e.g., an insulin cartridge), a dial or other mechanism to specify a dose, and a pen needle for transcutaneous delivery of the insulin into the tissue or vasculature of the person with diabetes. In a reusable insulin injection pen, the insulin container (e.g., the cartridge) is replaceable or refillable. A prefilled insulin injection pen is intended for use during a limited time. The dose-specifying mechanism can include a rotatable wheel coupled to mechanics and/or electronics for capping the administered amount of insulin at the volume of the specified dose (e.g., in terms of a number of units of insulin). The dose-specifying mechanism can have a mechanical and/or electronic display that reflects the current setting of the mechanism. The pen needle can be permanently attached to the housing of the insulin injection pen (e.g., as in the case with a disposable pen), or it can be removable (e.g., so that a new needle can be applied when needed). For example, a replaceable pen needle can include a hollow needle affixed to a fitting configured for removeable attachment to an end of the insulin injection pen.

Some examples herein refer to a mobile communication device. As used herein a mobile communication device includes, but is not limited to, a mobile phone, a smartphone, a wearable electronic device (e.g., a smart watch), a tablet, a laptop computer, a portable computer, and similar devices. A mobile communication device includes one or more processors, a non-transitory storage device (e.g., a memory and/or a hard drive) holding executable instructions for operating the mobile communication device, wireless communication components, and one or more input and/or output devices (e.g., a touchscreen, display, or keyboard). The mobile communication device can operate according to one or more application programs stored locally on the mobile communication device, or remotely (e.g., when using cloud computing), or combinations thereof. The mobile communication device can execute at least one operating system in order to perform functions and services.

Some examples herein refer to a blood glucose meter (BGM). A BGM is an electronic device configured to receive a sample from the person with diabetes (e.g., a blood sample) and analyze the sample to estimate the blood glucose level of the person with diabetes. The BGM may be configured to have a new test strip partially inserted into the BGM for each sample, and the person then places a drop of blood onto the end of the test strip extending from the BGM. The BGM performs testing on the drop of blood through the test strip.

Some examples herein refer to a continuous glucose monitor (CGM). A CGM is an electronic device configured to take readings of glucose values on an ongoing basis or at regular intervals in order to estimate the blood glucose level of the person with diabetes. Some CGMs determine blood glucose values periodically (e.g., after a certain number of seconds or minutes) and output the information automatically or upon being prompted. The CGM may include wireless communication components for one or more types of signaling, including, but not limited to, Near-Field Communication (NFC) and/or Bluetooth communication.

Some examples herein refer to estimated glucose value (EGV). EGV includes one or more values representing an estimate of the concentration of glucose in a person's blood. The estimate can be associated with a greater or smaller uncertainty (e.g., the error in the determination) and can be characterized as an approximation of the amount of glucose present per unit volume of blood. EGV can be determined or approximated by any of multiple different devices and/or techniques. For example, a CGM, a BGM, and/or another glucose sensor can generate an output that corresponds to EGV.

In some embodiments, systems, devices, and methods provided herein can recommend insulin doses (e.g., dosages of long-acting and/or rapid-acting insulin) using any suitable technique. In some embodiments, recommended insulin dosages may be based upon blood glucose data (e.g., current EGV from a CGM, flash glucose monitor, BGM, or any other sensor, blood glucose trend data, etc.), insulin administration data (bolus dosage amounts of rapid-acting insulin, dosages of long-acting insulin, dosage times, calculation of Insulin-on-Board ("IOB") and/or active insulin, etc.), meal data (mealtimes, user estimated carbohydrates, user estimated meal categorizations, user estimated glycemic impact of meal user meal history, user meal trends, etc.), and/or one or more insulin delivery parameters (e.g., total daily dose of basal insulin or long-acting insulin, carbohydrate-to-insulin ratio (CR), insulin sensitivity factor (ISF), etc.). Methods, devices and systems provided herein can, in some embodiments, adjust insulin delivery parameters over time based on glucose data and/or insulin administration data.

Some examples herein refer to long-acting insulin and rapid-acting insulin, or in some cases more generally to first and second types of insulin. Insulin used for therapeutic treatment is often synthesized human insulin. Moreover, different insulins can be characterized in how quickly they typically begin to work in the body of the person with diabetes after administration, and/or how long they typically remain active in the body of the person with diabetes. Rapid-acting insulin can be used to dose for meals or to correct high blood sugar. There is more than one type of insulin that can be considered a rapid-acting insulin. Many, but not necessary all, rapid-acting insulins begin working within about one hour after administration. Examples of rapid-acting insulin include, but are not limited to, insulin aspart (e.g., NovoLog^{®}, Fiasp^{®}, and NovoLog^{®} FlexPen^{®}), insulin lispro (e.g., Humalog^{®}, Admelog^{®}, and Humalog^{®} KwikPen^{®}), and insulin gluisine (e.g., Apidra^{®}, and Apidra^{®} SoloSTAR^{®} Pen). Similarly, there is more than one type of insulin that can be considered a long-acting insulin, and many, but not necessarily all, long-acting insulins begin working about one hour or longer after administration. Long-acting insulin is often referred to as basal insulin (e.g., insulin used to support basic metabolic needs). Generally, a long-acting insulin has a greater active time (i.e., the length of time that the insulin continues to be active in the body of the person with diabetes after administration) than a rapid-acting insulin. Examples of long-acting insulin include, but are not limited to, insulin glargine (e.g., Lantus^{®}, Toujeo^{®}, and Basaglar^{®}), insulin detemir (e.g., Levemir^{®}), and insulin degludec (e.g., Tresiba^{®}). As such, a long-acting insulin is an example of a type of insulin having an active time that is longer than an active time for a type of insulin such as a rapid-acting insulin.

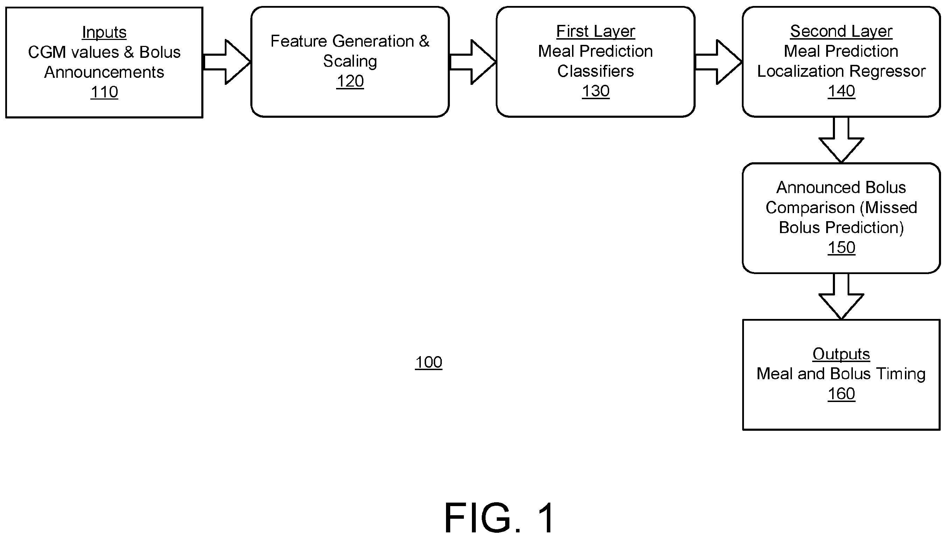

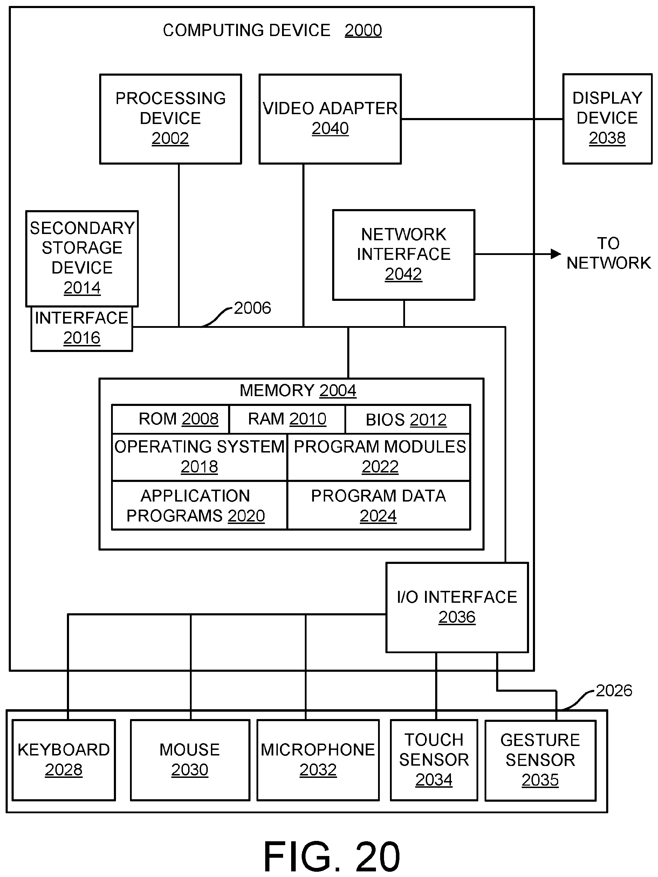

FIG. 1 shows an example of a system 100 that can detect a meal and/or a missed bolus. The system 100 can be used with one or more other examples described herein. The system 100 or any components thereof can be implemented using one or more computerbased modules, for example as described below with reference to FIG. 20.

The system 100 can use one or more inputs 110. In some implementations, the inputs 110 include one or more blood glucose values for the person with diabetes. For example, the blood glucose values can originate in a CGM or a BGM, and can be received in batch or substantially in real time. In some implementations, the inputs 110 include bolus information regarding the person with diabetes. For example, the bolus information can reflect actual detection of the time of administration of the bolus, actual detection of the amount of the bolus, estimation of the time of administration of the bolus (e.g., by way of a proxy such as detecting pen cap removal/replacement), and/or estimation of the amount of the bolus. One or more other inputs can be used in addition or instead.

The system 100 can use one or more feature components 120. In some implementations, the feature component 120 is configured for receiving some or all of the inputs 110 and generating one or more features therefrom. For example, any or all features described elsewhere herein can be generated in the system 100. In some implementations, the feature component 120 is configured for scaling one or more features from the inputs 110. For example, any or all features described elsewhere herein can be scaled in the system 100. In some implementations, scaling can involve calculating the mean and/or standard deviation for one or more of the features. For example, the calculated mean can be subtracted from the feature, and the difference can be used as a feature in addition to, or instead of, the originally calculated feature. For example, the feature can be divided by the calculated standard deviation, and the quotient can be used as a feature in addition to, or instead of, the originally calculated feature. Both of the above approaches can be used on the feature(s). In some implementations, scaling can be done to prevent a classifier or regressor from using the size of a particular feature as a proxy for information. For example, just because the feature has a large value does not mean the feature should be considered more important than another feature. In some implementations, a training data set is used for training classifier or regressor, and the mean and/or standard deviation can be determined for the training set for later use in testing and/or run time use.

The system 100 can use a first layer 130 of one or more machine-learning components. The first layer 130 can be characterized as including one or more meal prediction classifiers. The first layer 130 is sometimes referred to as a set of one or more classifiers. In some implementations, the first layer 130 includes one or more classifiers. For example, the classifier(s) can analyze the generated feature(s) and make a determination (e.g., as a binary outcome) of whether a meal has occurred at a given time. In some implementations, one or more of a Random Forest Classifier, a logistic regression classifier, a K-nearest neighbors classifier, an extra-trees classifier, and/or a gradient-boosted tree classifier can be used. In some implementations, one or more of a linear classifier, support vector machine, quadratic classifier, kernel estimator, a boosting classifier, a decision-tree classifier, a neural network, and/or a learning-vector quantization classifier can be used.

The data in the inputs 110 can be organized according to one or more measures of time, and the first layer 130 can operate accordingly. In some implementations, the inputs 110 can include discrete values associated with respective time intervals, sometimes referred to as time points. Any length of time interval can be used. For example, each value can be associated with a respective 15-minute or 5-minute time interval or time point (e.g., based on the sampling frequency of a CGM). The first layer 130, moreover, can generate one classification event (e.g., an outcome reflecting a classification of meal or no meal) for each classifier for each time point. For example, the first layer 130 can predict that no meal occurred at a particular time point even if the bolus information indicates (e.g., by a pen cap removal) that a bolus was injected at that time point.

The first layer 130 can use data pertaining to multiple time points to make the classification for each time point. In some implementations, for each time point the classifier(s) can take into account the data from a time interval (e.g., one hour of future, or later-detected, data) in making its classification. For example, this can account for the fact that the physiological effect of carbohydrate consumption is not instantaneous but rather can be distributed over time. As a result the classifier(s) of the first layer 130 can likely detect multiple meal events in nearby (e.g., adjacent) time points. Moreover, if multiple classifiers are used in the first layer 130, each one can potentially give a different (e.g., slightly different) result based on substantially the same input data. For example, aggregation of the results of multiple classifiers can then be performed, such as along the lines exemplified later in this description.

The system 100 can use a second layer 140 of one or more regressors. The second layer 140 can be characterized as including one or more meal prediction localization regressors. The second layer 140 is sometimes referred to as a set of one or more regressors. For example, the meal prediction localization regressors can seek to determine exactly when the meal predicted by the first layer 130 occurred. In some implementations, the second layer 140 includes one or more regressors. For example, the regressor(s) can analyze the output of the classifier(s) in the first layer 130 and make a determination (e.g., as a binary outcome) of whether a meal has occurred at a given time. In some implementations, the second layer can be based on a standard logistic function, such as a sigmoid function, that takes a real input and outputs a value between zero and one, which can be interpreted as the determined probability that a meal occurred. For example, a Random Forest-based regressor can be used. The second layer 140 can take the output of the classifier(s) in the first layer 130, and also one or more of the inputs 110, and convert them to a single point (i.e., a single time point) that the second layer 140 considers to be the beginning of the meal detected by the first layer 130. That is, the second layer 140 can provide one result per time interval, wherein the time interval is longer that the time periods considered by the first layer 130. For example, the second layer 140 can generate one result per hour of data when the first layer 130 gives output for each 15-minute time point. In some implementations, the output of the second layer 140 can be in form of one or more values. For example, a zero output can correspond to determination of a meal beginning in the first 15 minutes of the hour, a one output can correspond to determination of a meal beginning in the second 15 minutes of the hour, and so on. In the just described example, a negative value in the output of the second layer 140 can correspond to no meal being detected. The output of the second layer 140 (e.g., integer values) can be converted into a time lap of Boolean values (e.g., by the system 100 or another system). For example, a false or zero value can represent no meal event for the time point, and a true or one value can represent a meal event for the time point.

The system 100 can include an evaluation 150. In some implementations, the evaluation 150 can be characterized as a comparison of the outcome(s) of the second layer 140 with one or more other things (e.g., with at least one of the inputs 110). For example, the evaluation 150 can be characterized as an announced-bolus comparison. In some implementations, the evaluation 150 can correspond to a prediction regarding one or more characteristic relating to the person with diabetes. For example, the evaluation 150 can generate a prediction that the person missed a bolus; that is, that the person did not timely receive insulin for an ingested meal. In some implementations, the meal prediction(s) of the second layer 140 can be directly compared with the bolus information of the inputs 110. Any detected meal that does not have a corresponding detected bolus event within a predetermined time period (e.g., on the order of one hour) can be declared as a missed bolus event; that is, as a missed bolus.

The system 100 can generate one or more outputs 160. The outputs 160 can be the direct result of the evaluation 150. In some implementations, the outputs 160 can be a time map that represents detected events (e.g., any missed boluses). For example, the outputs 160 can be a time map where a true or one value can represent a missed bolus for the time point corresponding to the estimated time of the meal, and a false or zero value can represent no missed bolus for the time point.

Operation of the system 100 can illustrate performance of a computer-implemented method of detecting a missed bolus. Such a method can include receiving blood glucose values (e.g., in the inputs 110) and bolus information (e.g., in the inputs 110) regarding a person with diabetes, the blood glucose values and the bolus information relating to a period of time. The method can include classifying (e.g., using the first layer 130), based on the blood glucose values and the bolus information, each of multiple time periods within the period of time regarding whether the time period is associated with ingestion of a meal by the person. The method can include performing regression analysis (e.g., by the second layer 140) on the classified multiple time periods to identify a first time period of the classified multiple time periods as corresponding to a beginning of the meal. For an identified time period, the bolus information can indicate (e.g., as indicated by the evaluation 150) no bolus associated with the identified first time period. The method can include associating (e.g., in the outputs 160), based on the regression analysis, a missed bolus event with the meal. One or more actions can be performed based on the missed bolus event having been associated with the meal. For example, the action can include issuing an alert regarding the person.

The system 100 can be included in one or more devices or other apparatuses. In some implementations, the system 100 is included in an insulin treatment device. For example, the insulin treatment device can be, or include, a smartphone, a pen cap for an insulin pen, an insulin pen, an insulin pump, a CGM, or a BGM. The system 100 can have a user interface and an alert regarding the person can be issued or generated using the user interface, the alert being based on the missed bolus event being associated with the meal.

The system 100 was tested using simulated data for a fictitious yet statistically representative population. The results represent both the ability to predict meals and to detect missing boluses. For both meal prediction and missed boluses, the results are characterized with regard to both precision and recall, in terms of positive values and negative values. For meal prediction, a positive value means that a meal was predicted at a certain time point, and a negative value means that a meal was not predicted at the certain time point. For missed bolus, a positive value means that a missed bolus was predicted at a certain time point, and a negative value means that a missed bolus was not predicted at the certain time point. Precision is defined as the ratio between the number of true positive values and the sum of true and false positive values. That is, the precision answers the question: Of all values (e.g., time points) that the system 100 labeled as true (e.g., as being a meal, or as being a missed bolus), how many were actually true? Recall is defined as the ratio between the number of true positive values and the sum of true positive values and false negative values. That is, the recall answers the question: Of all values (e.g., time points) that the system 100 should have labeled as true, how many were correctly labeled? The following table shows examples of results:

| **Description** | **Meal Prediction** | | **Missed Bolus** | |
|---|---|---|---|---|
| | **Precision** | **Recall** | **Precision** | **Recall** |
| Baseline (10% missed bolus, 0.3 noise factor, early and late bolusing enabled) | 0.961 | 0.952 | 0.751 | 0.720 |
| 10% missed bolus, 0.4 noise factor, 2x meal STD, early and late bolusing enabled | 0.951 | 0.945 | 0.675 | 0.679 |
| 10% missed bolus, 0.5 noise factor, all boluses same time as carbs | 0.939 | 0.952 | 0.548 | 0.724 |

Here, all of the test runs used a standard chance of a missed bolus (e.g., 10%). To simulate very large and very small meals (e.g., a snack), the meal standard deviation (STD) was doubled in the second test run. The noise factor was increased from each test run to the next. The results show that increasing the noise factor can significantly increase the false positive rate for missed boluses, and thereby decrease the precision.

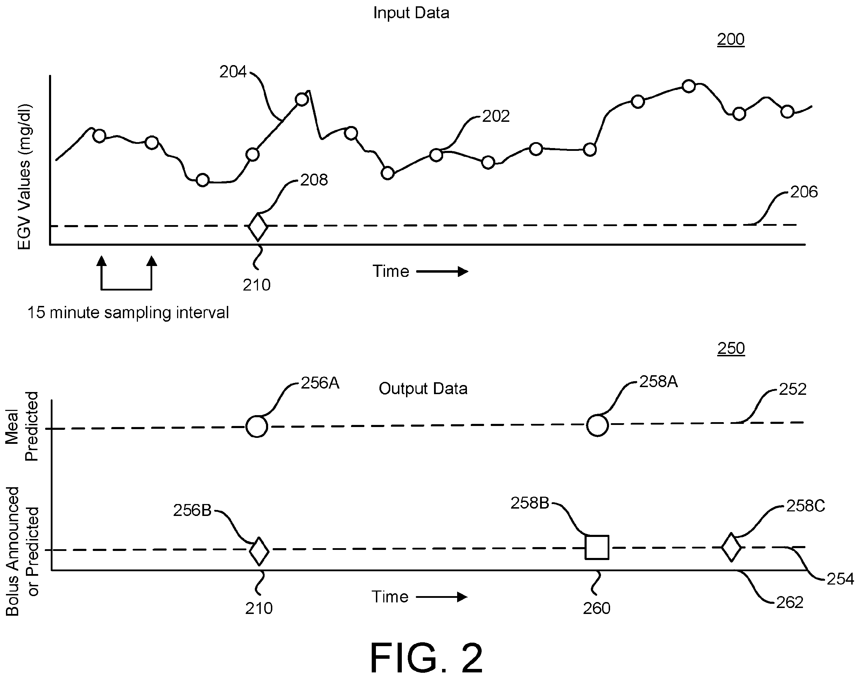

FIG. 2 shows an example of input and output for meal and/or missed-bolus detection. Here, the input includes input data 200 and the output includes output data 250. The input data 200 and/or the output data 250 can be used with (e.g., generated by and/or used by) one or more systems or devices described elsewhere herein. For example, the input data 200 can be, or be part of, the inputs 110 (FIG. 1) and the output data 250 can be, or be part of, the outputs 160 (FIG. 1).

The input data 200 is here presented as a graph in a coordinate system having a blood-related value (e.g., EGV, such as in units of milligrams/deciliter (mg/dl)) on the vertical axis, and having time (e.g., in time points of a predefined sampling interval, such as 15 minutes) on the horizontal axis. The input data 200 here includes multiple sampled values 202, which are illustrated as circles. For example, each of the sampled values 202 corresponds to an EGV estimation performed by a CGM. The graph also shows, for purpose of clarity, multiple true values 204, which are illustrated as a continuous line that connects respective ones of the sampled values 202 to each other. The true values 204 may not be part of the input data 200; rather, the true values can represent the actual EGV values of the person with diabetes if determined continuously. That is, the sampled values 202 represent periodic samplings of the true values 204. The input data 200 here includes a line 206 corresponding to one or more announced boluses. For example, an entry 208 on the line 206 indicates that a bolus was announced as being injected at a time 210. Because carbohydrates affect blood glucose values over a finite time following ingestion of a meal, and because administered insulin can act on a shorter time scale (e.g., rapid-acting insulin) or a longer time scale (e.g., long-acting insulin), the system can wait at least a predetermined amount of time after the detected beginning of a meal before making a determination whether a bolus is missed or is injected late relative to the meal.

The output data 250 is here presented as a diagram in a coordinate system having predictions made or announcements detected on the vertical axis, and having time (e.g., in time points of a predefined sampling interval, such as 15 minutes) on the horizontal axis. The output data 250 here includes a line 252 corresponding to prediction of one or more meals, and a line 254 corresponding to announcements of one or more injected boluses, or a predicted bolus. For example, an entry 256A on the line 252 indicates that a meal has been predicted as beginning at the time 210. Moreover, an entry 256B on the line 254 corresponds to the entry 208 in the input data 200 and indicates that a bolus was announced. Because the entry 256B is sufficiently contemporaneous with the entry 256A (e.g., coincident, or at least within the same hour), the bolus associated with the entry 256B can be considered timely with regard to the meal associated with the entry 256A. That is, by the entry 256B being associated with the entry 256A, the system can decide not to associate the corresponding meal with a missing bolus event or a late bolus event.

Here, the output data includes an entry 258A on the line 252 that corresponds to another prediction of a meal. On the line 254, however, there is no entry corresponding to the entry 256B discussed above, meaning that the input data 200 does not contain a bolus announcement corresponding to a time 260. Rather, an entry 258B which occurs on the line 254 at the time 260 can represent a predicted bolus and/or a determined missed bolus. For example, based on determining the entry 258A (i.e., a predicted meal) the entry 258B can represent an expectation by the system that a bolus should be announced in connection (e.g., coincident, or at least within a predefined time) with the detected meal. As another example, after the meal is detected and it is determined that no bolus announcement corresponds to the meal, the entry 258B can represent the determination that a bolus was missed for the detected meal. That is, when bolus information in the input data 200 includes no bolus separated from the time period of the entry 258A by at most a predefined time, this can trigger a determination of no bolus being associated with the identified time period. The determination of a missed bolus can trigger performance of one or more actions, including, but not limited to, issuance of an alert or a recommendation, or an automatic bolus injection, for example as described elsewhere herein.

A determination of a missed bolus can be the basis for issuing a communication to or for the benefit of the person with diabetes regarding their therapy, which communication can sometimes be referred to as an "insight". The insight can be communicated to the person directly (e.g., by way of a controller or other electronic device) and/or indirectly (e.g., by way of sending the insight to the person's health care provider to serve as a basis for coaching the person). In some implementations, an insight can indicate that the person misses meal boluses on a regular basis. For example, after analysis of about one week of data or more, a system can generate an insight communicating that the person appears to have missed, say, three boluses during that time. As another example, the insight can convey that the person appears to miss morning boluses on a regular basis.

The output data 250 can indicate one or more late boluses. In some implementations, an entry 258C on the line 254 can indicate that a bolus announcement was made at a time 262 (the bolus announcement not shown in input data 200 for simplicity). However, the input data 200 may not have predicted any meal as occurring within a predetermined time (e.g., one hour) of the time 262. Rather, the entry 258C can therefore be deemed to be a late bolus injection for a meal that occurred sometime earlier. Here, for example, the entry 258C can be considered a late bolus event for the meal corresponding to the entry 258A. That is, when bolus information in the input data 200 does include a bolus separated from the time period of the entry 258A by more than a predefined time, then a late bolus event can be associated with the announced bolus. The determination of a late bolus event can trigger performance of one or more actions, including, but not limited to, issuance of an alert or a recommendation, for example as described elsewhere herein.

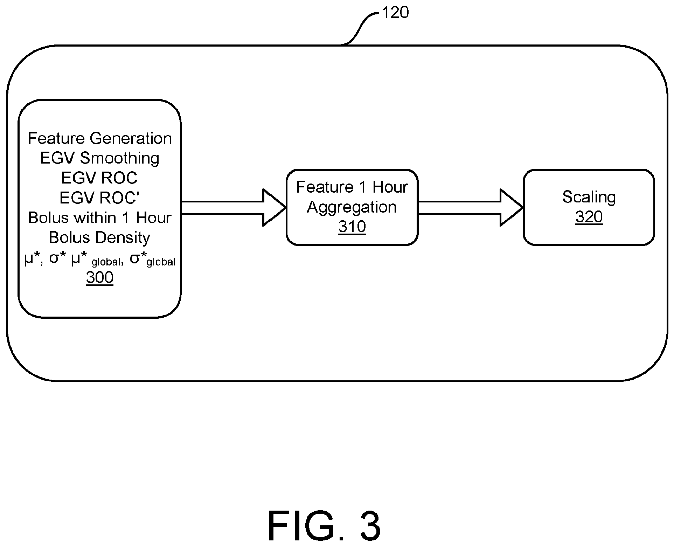

FIG. 3 shows an example of feature generation, and aggregation and scaling of one or more features. The features can be managed using the feature component 120, for example as part of the system 100 (FIG. 1). The feature component 120 can include a feature generation component 300. In some implementations, the feature generation component 300 can generate one or more features based on the inputs 110 (FIG. 1). For example, the feature generation component 300 can generate a feature corresponding to a smoothed EGV, a feature corresponding to a rate of change (ROC) of EGV, a feature corresponding to a derivative (ROC') of the ROC of the EGV, a feature corresponding to whether a bolus is detected within a predetermined time period (e.g., one hour) of a detected beginning of a meal, a bolus density (e.g., a bolus probability by time of day), a geometric mean of the next hour of data (sometimes referred to as µ*), a geometric standard deviation of the next hour of data (sometimes referred to as σ*), a geometric mean of the entire iteration of data (sometimes referred to as µ*_{global}), and/or a geometric standard deviation of the entire iteration of data (sometimes referred to as σ*_{global}). The µ and σ features can be used to give the system a sense of how the local (e.g., the hour of interest) data differs from the global data (i.e., the entire iteration). In some implementations, the system can operate based on the assumption that, on average, after a meal the person with diabetes will have a higher µ* and σ* since the blood glucose should be increasing rapidly within the one-hour period. This can be compared to the average (global) values across the entire iteration for reference. As another example, a difference between local and global values can be used. Other features can be generated and/or used.

The feature component 120 can include a feature aggregation component 310. In some implementations, the feature aggregation component 310 aggregates one or more features over a predetermined time period (e.g., by also taking into account future and/or past feature values). For example, the feature aggregation component 310 can aggregate the next one-hour time period's worth of feature values at each time point. Other aggregations can be used.

The feature component 120 can include a feature scaling component 320. The feature scaling component 320 can seek to bring a feature away from large numerical values to avoid giving undue significance to that feature. For example, the feature scaling component 320 can subtract the mean of the (aggregated) feature from the feature value(s) and/or can divide the (aggregated) feature value(s) with the standard deviation of the feature values.

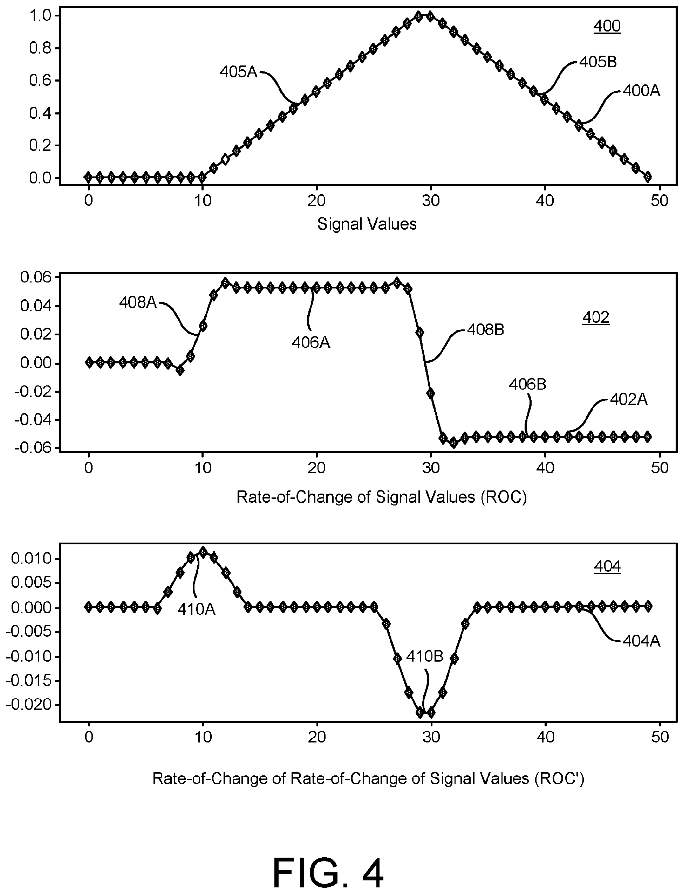

FIG. 4 shows an example of features 400, 402, and 404 relating to EGV and rates of change. The features 400, 402, and 404 can be used with one or more other features described herein. The features 400, 402, and 404 can be used with one or more systems or devices described elsewhere herein. Each of the features 400, 402, and 404 is presented as a series of data points 400A, 402A, and 404A, respectively, which represent values of the feature on a vertical axis associated with time measured against a horizontal axis. The data points 400A, 402A, and 404A can be connected by a continuous line, which may be an interpolation performed on the input data or on data calculated from the input data. In some implementations, a linear interpolation of the respective data points 400A can be performed though any time points in the feature 400 that are not associated with values. For any remaining time points in the feature 400 that are not associated with values, they can instead be associated with the mean of the signal values. In some implementations, a linear interpolation can be performed for any missing data except missing data at the beginning or end of a sequence of data (e.g., one week of data). For example, the interpolation may require data to be present at the beginning and end of the interval of missing data. In contrast, for one or more missing beginning or end data points, a substitute value can be used. For example, the global mean of the values in the sequence of data can be used. In some implementations, the substitute value can include a mean of values that are local to the missing data.

In some implementations, the feature 400 can be characterized as signal values and may be present (e.g., as raw data) in an input signal. For example, the feature 400 can correspond to EGV for a person with diabetes (e.g., obtained from a CGM). Here, the feature 400 includes an upward slope 405A followed by a downward slope 405B. The feature 400 can be smoothed in one or more processing steps. In some implementations, the smoothing can involve performing a convolution on at least part of the feature 400. For example, a Savitsky-Golay filter can be applied.

In some implementations, one or more other features (e.g., the feature 402 and/or 404) can be derived from input data, such as from the feature 400. Such feature(s) can be derived to obtain more informative and/or more convenient representations of data. In some implementations, the feature 402 can correspond to a ROC of the signal values (e.g., a derivative of the EGV). Here, the feature 402 includes a region 406A of positive values corresponding to the upward slope 405A, and a region 406B of negative values corresponding to the downward slope 405B. Here, the feature 402 includes an upward slope 408A and a downward slope 408B.

In some implementations, the feature 404 can correspond to a ROC of the ROC of the signal values (e.g., a second derivative of the EGV). Here, the feature 404 includes a region 410A of positive values corresponding to the upward slope 408A, and a region 410B of negative values corresponding to the downward slope 408B.

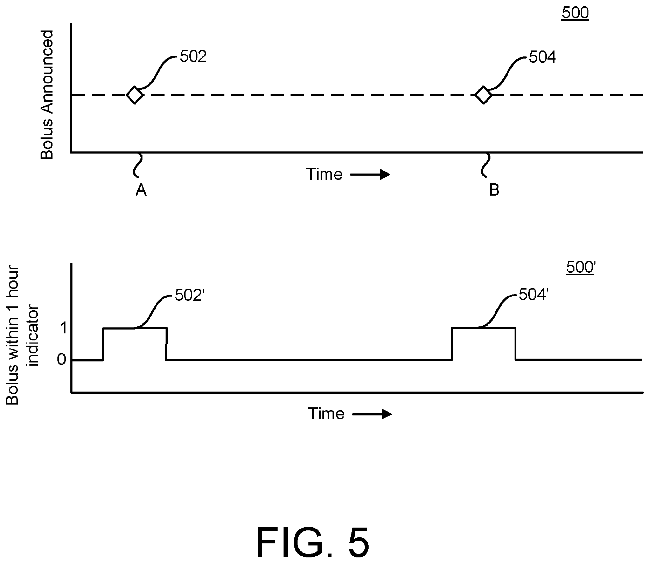

FIG. 5 shows an example of spreading out data 500 of a feature. The data 500 can be used with one or more other features described herein. The data 500 can be used with one or more systems or devices described elsewhere herein. The data 500 includes data points 502 and 504 corresponding to feature values indicated against a vertical axis and associated with time points along a horizontal axis. In some implementations, the data points 502 and 504 correspond to bolus announcements. For example, each of the data points 502 and 504 can represent a detection or determination that a person with diabetes injects a bolus of insulin.

The data point 502 is here positioned at a time point A, and the data point 504 at a time point B, on the time axis. In some implementations, the data points 502 and 504 can have essentially no extension in time (that is, in the horizontal direction in the present illustration). Rather, each of the data points 502 and 504 can be characterized as a "spike" and can correspond to a delta function as follows: $data point 502 = δ \left(t-A\right) ,$ and $data point 504 = δ \left(t-B\right) ,$ where t is the measure of time and *δ* is the Dirac delta function.

A feature relating to boluses (e.g., bolus announcement) can be used to give some information on when boluses actually occurred. However, instead of directly using the spikes corresponding to the data points 502 and 504 in the raw data as a feature, one can make the bolus information less informative in one or more ways. In some implementations, the bolus information can be broadened to have a finite time duration. Here, data 500' results from such broadening and includes a shape 502' corresponding to the data point 502, and a shape 504' corresponding to the data point 504. In some implementations, the shape 502' and/or 504' is a rectangle. For example, the shapes 502' and/or 504' can be formed by convolving the respective delta function with a rectangle function. The shape 502' can be centered at the time A and the shape 504' can be centered at the time B. In some implementations, each of the shapes 502' and/or 504' can extend for a predetermined time. For example, the shape 502' can extend for one hour before, and one hour after, the time A, for a spreading of the bolus information over two hours in this example. Similarly, the shape 504' can be spread over a predetermined time, such as two hours. The predetermined time can be a multiple of a time relevant to insulin treatment. In some implementations, the predetermined time over which bolus information is spread can be a multiple of the maximum delay between the beginning of a meal and administration of the corresponding bolus. For example, when the diabetes management system waits one hour after the beginning of a meal until determining whether a bolus is missing, then the duration of the shape 502' and/or 504' can be a number of hours (e.g., two hours). Temporal broadening or spreading of bolus information can have advantages in data analysis. Without broadening, a classifier may excessively rely or only rely on the bolus information (e.g., the presence of a bolus announcement) to predict when meals occurred, thereby missing (i.e., failing to predict) every meal that does not have an associated bolus announcement. In an implementation that at least in part seeks to detect missed boluses, such a result would defeat the purpose of the detector. Therefore, to decrease the chance of such overfitting, the bolus information can be made less informative (without omitting the bolus information), so that the classifier(s) will take into account also other features.

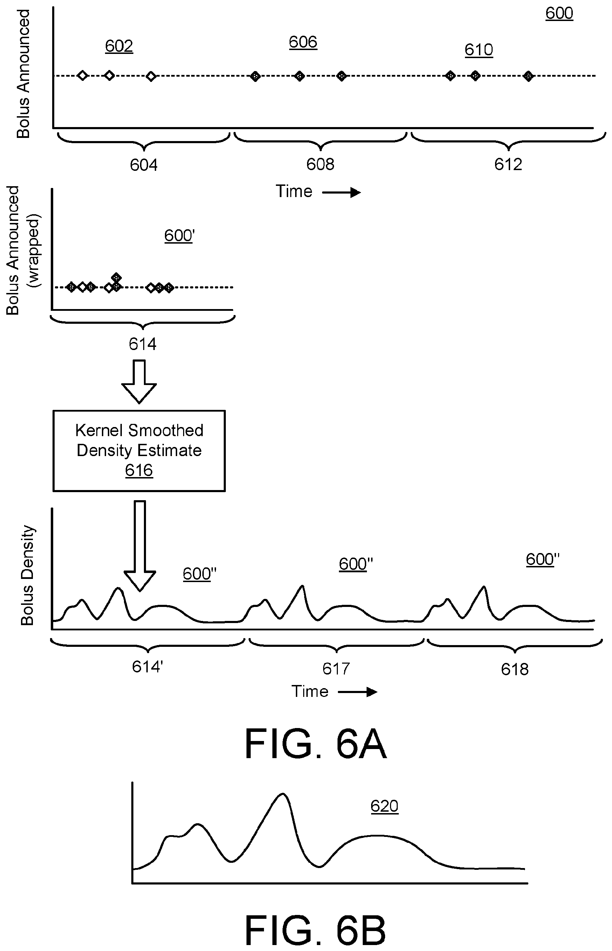

FIG. 6A schematically shows an example of aggregating bolus information 600. The information 600 can be used with one or more other features described herein. The information 600 can be used with one or more systems or devices described elsewhere herein. The information 600 includes data points corresponding to feature values indicated against a vertical axis and associated with time points along a horizontal axis. Particularly, the information 600 includes data points 602 within a time interval 604, data points 606 within a time interval 608, and data points 610 within a time interval 612. In some implementations, the data points 602, 606, and 610 correspond to bolus announcements. For example, each of the data points 602, 606, and 610 can represent a detection or determination that a person with diabetes injects a bolus of insulin. The time intervals 604, 608, and 612 can be of equal length (e.g., 24 hours each). That is, the period of time to which the information 600 pertains (e.g., a number of days) can be subdivided into shorter time intervals such as the time intervals 604, 608, and 612.

A wrapping of the information 600 can be performed. In some implementations, this involves wrapping the bolus events corresponding to the data points 602, 606, and 610 over a shorter time interval, such as one of the time intervals 604, 608, and 612. Here, information 600' (shown in a separate diagram for clarity), includes the data points 602, 606, and 610 wrapped over a time interval 614, so that the data points 602, 606, and 610 are distributed over the time interval 614. The time interval 614 has the same length as each of the time intervals 604, 608, and 612 (e.g., 24 hours). This can be considered an aggregation of bolus information, for example so that bolus events are aggregated. The information 600' gives an estimate of when, throughout the course of a predetermined time interval (e.g., a 24-hour period), the bolus events tend to occur, on average.

The information 600' can be subjected to one or more kinds of processing. In some implementations, a smoothing and/or estimation can be performed. For example, a kernel smoothed density estimate 616 can be performed on the bolus information (e.g., bolus events) in the information 600'. The kernel smoothed density estimate 616 can estimate, based on the information 600', the probability density function for the bolus events represented by the data points 602, 606, and 610. The outcome of performing the kernel smoothed density estimate 616 can be information 600" in its leftmost instance in the illustration, which is a continuous distribution of likelihood over a time interval 614'. The time interval 614' here corresponds to the time interval 614 for the information 600', and the information 600" corresponds to a continuous probability distribution that is based on the bolus events in the information 600'. The information 600" can be characterized as bolus density by time interval (e.g., bolus density by day), and can be used for one or more purposes. The information 600" can be a likelihood vector of predetermined length (e.g., 24 hours). The bolus density of the information 600" represents the estimated likelihood, at any time point, that the person with diabetes will inject insulin. For example, bolus density can be used as a proxy for meal occurrence likelihood. In some implementations, another operation than the kernel smoothed density estimate 616 can be performed on the information 600' to generate the information 600". For example, a filtering of the information 600' can be performed.

The information 600" can be replicated one or more times to extend the continuous distribution of likelihood over a longer time. Here, the leftmost instance of the information 600" is replicated into a time interval 617 that is adjacent the time interval 614', and also into a time interval 618 that is adjacent the time interval 617. As such, three instances of the information 600" are shown. For example, this replication shows an estimate of bolus density over a three-day period. Any number of replications can be performed.

A meal probability density can be similar or identical to a bolus density for the same person with diabetes. FIG. 6B shows an example of a meal probability density 620. The meal probability density 620 is a continuous probability distribution that can be based on the information 600', or directly on the bolus events in the information 600'. For example, based on blood glucose values and bolus information in input data, the meal probability density 620 for the person can be generated. The meal propensity of the meal probability density 620 represents the estimated likelihood, at any time point, that the person with diabetes will ingest a meal. The meal probability density 620 can be used for one or more purposes. In some implementations, the meal probability density 620 can be used in an estimation of whether the person with diabetes will ingest, or has recently ingested, a meal. For example, the determination whether the person has missed a bolus can be based at least in part on the determination, using the meal probability density 620, that the person has ingested a meal.

As mentioned, an action (e.g., alerting for a missed bolus) can be generated based on at least one threshold being met. Such threshold(s) can be dynamically adjusted based on the meal probability density 620. In some implementations, the threshold can be adjusted based on whether the value of the meal probability density 620 at the current time is relatively high or relatively low. For example, a relatively lower threshold for the alert can be used when the meal probability density 620 indicates that a current meal probability is relatively higher. As another example, a relatively higher threshold for the alert can be used when the meal probability density 620 indicates that a current meal probability is relatively lower.

Generating the meal probability density 620 can involve using a bolus density as a proxy for meal occurrence likelihood. After the information 600" is generated, the meal probability density 620 can be obtained from the information 600". As another example, after the meal probability density 620 is generated, the information 600" can be obtained from the meal probability density 620.

The above examples illustrate performance of a method of determining a personalized meal propensity. Such a method can include receiving bolus information (e.g., the data points 602, 606, and 610) regarding a person with diabetes, the bolus information comprising bolus events distributed within a period of time (e.g., three days). The method can include wrapping the bolus information over a time interval (e.g., the time interval 614) shorter than the period of time so that the bolus events are distributed within the time interval (e.g., as information 600'). The method can include generating a meal probability density (e.g., the meal probability density 620) based on the wrapped bolus information, wherein the meal probability density is a continuous function of time over the time interval. Such a method can be performed in or by one or more devices or other apparatuses. In some implementations, the method is performed in an insulin treatment device. For example, the insulin treatment device can be, or include, a smartphone, a pen cap for an insulin pen, an insulin pen, an insulin pump, a CGM, or a BGM.

Bolus information can include amount information about the boluses. For example, the person with diabetes can input the amount information into the system (e.g., a smartphone). As another example, an insulin treatment device can have a dose capture function that registers or estimates the bolus amount and provides that information to one or more other components. The information 600" can then additionally indicate the likely amount of the bolus, in addition to the likelihood of a bolus being administered. Based on input data (e.g., blood glucose values and bolus information) and the meal probability density 620, an expected bolus amount can be determined. For example, based on knowing that the person with diabetes usually has a meal at a time of T hours in the day, and that the bolus amount is usually B units of insulin for that meal, and that the current time is approximately T hours, an expected bolus amount of B units can be determined. The bolus information regarding the currently (or most recently) administered bolus amount can then be compared to the expected bolus amount. One or more actions can be taken based on the outcome of such a comparison. For example, an alert can be issued regarding the person (e.g., substantially in real time) based on a discrepancy between the amount information and the expected bolus amount.

Information about administered boluses can be used in one or more types of insulin treatment systems. In an automated system that controls an insulin pump (e.g., a closed-loop insulin therapy system), such bolus information can be used in calculating a bolus amount to be automatically administered to the person by the system. For example, the bolus can be administered based on detecting a rise in blood glucose level at a time when the person normally or usually eats, yet the system is not detecting a request for a meal bolus at that time. In an insulin therapy system that does not have automatic bolusing (e.g., a system where the person is responsible for manually injecting themselves with insulin using an insulin pen or other device), one or more insights can be generated. In some implementations, an insight can be triggered by detection of a meal of a size that is out of the ordinary (e.g., abnormal) for the person. For example, the insight can communicate that the person tends to have poorly managed blood glucose levels whenever they ingest a relatively small or relatively large meal. In some implementations, an insight can be triggered by detection of falling glucose levels. For example, the insight can communicate a message along the lines of "Today at lunch you only bolused 3 units; you normally bolus 10 units and you currently have an elevated blood glucose level." As another example, the insight can communicate a message along the lines of "Your blood glucose levels are rapidly falling and you bolused 10 units of insulin, more than twice your normal lunch time insulin; consider treatment options." Other insights and/or conditions for triggering an insight can be used.

More than one version of the meal probability density 620 can be generated. In some implementations, the meal probability density 620 can be specific to one or more particular days of the week for the person with diabetes, and one or more other meal probability densities can then be generated for the other day(s) of the week. For example, the person may have a meal probability density for weekdays that differs from a meal probability density for weekend days. In some implementations, one or more thresholds for a feature in a machine-learning classifier (e.g., in the first layer 130 of FIG. 1) can be changed based on the meal probability density 620. For example, the classifier(s) can be configured to more readily classify a time point as being a meal if the meal probability density 620 indicates a higher likelihood that the person with diabetes ingests meals at the time point. As another example, the meal probability density 620 can be taken into account in insulin therapy management by reducing a threshold for determining that a bolus was missed; that is, for associating a missed bolus event with a detected meal.

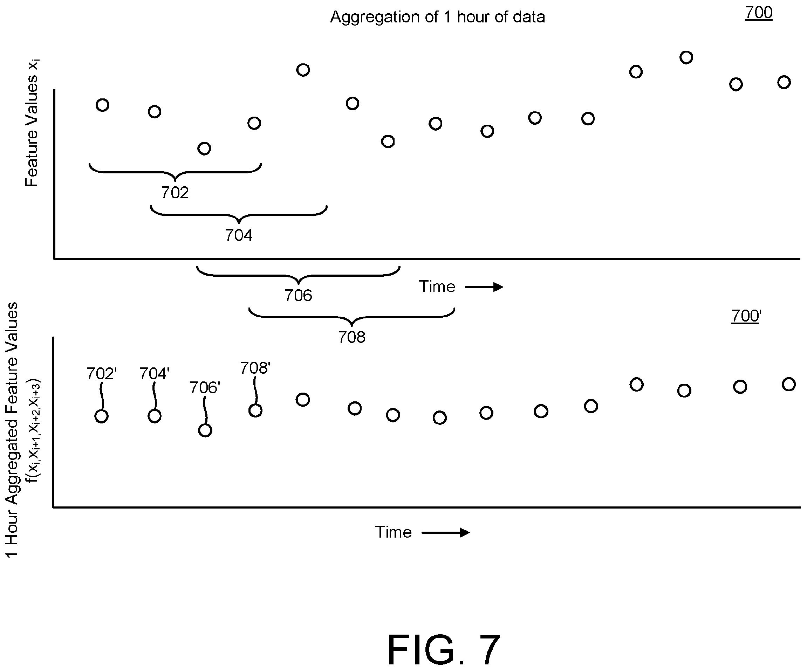

FIG. 7 schematically shows an example of aggregating feature information 700 into aggregated feature information 700'. The feature information 700 and/or the aggregated feature information 700' can be used with one or more other features described herein. The feature information 700 and/or the aggregated feature information 700' can be used with one or more systems or devices described elsewhere herein.

The aggregated feature information 700' can be formed from the feature information 700. The formation can involve aggregation over a period of time, or a fixed number of samples, to name just two examples. In some implementations, the feature information 700 can be labeled as individual data points Xᵢ, where *i* is an index. The individual feature values in the feature information 700 can include any type of feature, including, but not limited to, blood glucose values and/or bolus information. For every data point Xᵢ in the feature information 700, the aggregated value in the aggregated feature information 700' can be labeled f(Xᵢ, Xᵢ₊₁,, Xᵢ₊₂, Xᵢ₊₃), indicating that in this example each aggregated data point Xᵢ' in the aggregated feature information 700' is a function of (e.g., an aggregation of) four data points from the feature information 700, labeled Xᵢ, Xᵢ₊₁,, Xᵢ₊₂, Xᵢ₊₃, respectively. As such, a data point 702' in the aggregated feature information 700' can be formed as an aggregation of a group 702 of data points in the feature information 700. Similarly, a data point 704' in the aggregated feature information 700' can be formed as an aggregation of a group 704 of data points in the information 700, a data point 706' in the aggregated feature information 700' can be formed as an aggregation of a group 706, and a data point 708' can be formed as an aggregation of a group 708. In this example, each of the data points 702' through 708' is an aggregation of the future hour of feature data from the feature information 700. The aggregated feature information 700' can be provided, alone or in combination with other information, to one or more classifiers (e.g., in the first layer 130 in FIG. 1). Two or more aggregations can overlap with each other. For example, each of the groups 702 through 708 includes at least one data point that is also included in another one of the groups 702 through 708. Other ranges and/or approaches for aggregation can be used. For example, a longer or shorter aggregation interval can be used. As another example, data points earlier in time (i.e., in the past) and/or later in time (i.e., in the future) can be aggregated. The terms "past" and "future" are here used relative to the point in time of the aggregated feature information (i.e., the time to which the information is being aggregated).

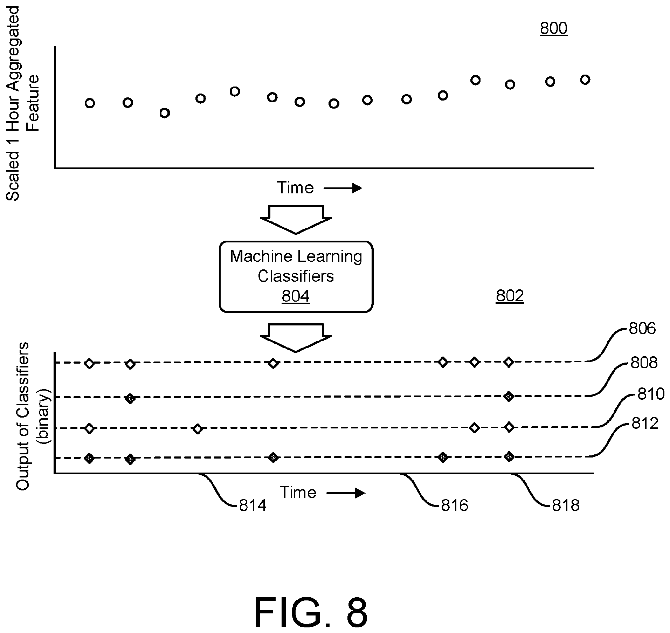

FIG. 8 schematically shows an example of classification based on a feature. Here, feature information 800 will be used for generating classifier output 802 by feeding into one or more machine-learning classifiers 804 (e.g., in the first layer 130 of FIG. 1). The feature information 800 is indicated as a value (e.g., a scaled one-hour aggregated feature) measured against a vertical axis as a function of time measured against a horizontal axis. The feature information 800 and/or the classifier output 802 can be used with one or more other features described herein. The feature information 800 and/or the classifier output 802 can be used with one or more systems or devices described elsewhere herein.

In some implementations, the one or more machine-learning classifiers 804 are configured to predict, based on the feature information 800, when the person with diabetes ingests a meal. Each classifier can make a binary output for one or more points in time. In some implementations, the machine-learning classifiers 804 make outputs (e.g., a determination of meal or no meal) for every point in time. Any time interval between determinations can be used. For example, the machine-learning classifiers 804 can make a determination for every 15 minutes of the feature information 800. The outputs of the machine-learning classifiers 804 can be separate from each other in the classifier output 802. For example, each of lines 806, 808, 810, and 812 here corresponds to an output of a different classifier, which classifiers will here be referred to as classifiers A, B, C, and D, respectively. On each of the lines 806 through 812, one or more meal determinations can be indicated.

The machine-learning classifiers 804 may arrive at different conclusions regarding one or more time points. Here, for example, at a time 814 the classifier C, but none of the classifiers A, B, or D, determines that a meal takes place. At time 816, moreover, all of the classifiers A-D indicate that no meal is being ingested. At a time 818, finally, all of the classifiers A-D indicate that a meal is being ingested. Accordingly, there can be zero or more classifier outputs for each time point. Also, the machine-learning classifiers 804 can tend to classify multiple consecutive time points as being meals. In some situations, this can be because the person with diabetes ingested the meal over an extended time. In other situations, however, this can be caused by the relatively slow impact of carbohydrates on blood glucose levels, and/or by the relatively fast or slow action by the administered insulin. In some implementations, regression analysis (e.g., by the second layer 140 in FIG. 1) can be applied to such classifier output in an attempt to identify the beginning of a meal.

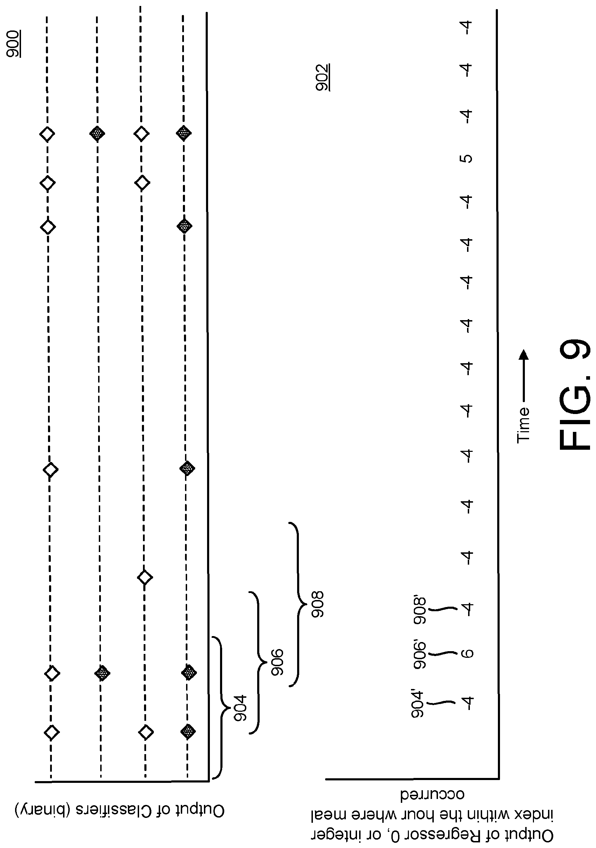

FIG. 9 schematically shows an example of regression based on classification. The regression takes classifier output 900 and uses it (e.g., by the second layer 140 in FIG. 1) to generate regressor output 902. The classifier output 900 is indicated as individual binary values (e.g., determinations of meal or no meal by individual classifiers) as a function of time measured against a horizontal axis. The feature classifier output 900 and/or the regressor output 902 can be used with one or more other features described herein. The classifier output 900 and/or the regressor output 902 can be used with one or more systems or devices described elsewhere herein.

An individual output in the regressor output 902 can be based on a range (or window) of the classifier output 900. Here, an output 904' in the regressor output 902 is based on a range 904 of the classifier output 900. Similarly, an output 906' is based on a range 906, and an output 908' is based on a range 908. In some implementations, the range can be about three hours. Other (shorter and/or longer) ranges can be used. Two or more of the ranges 904-908 can overlap with each other. The range(s) can have any of multiple temporal relations to the regressor value being determined. Here, the output 904' represents the center hour (e.g., middle hour among three hours) of the range 904. Similarly, the output 906' represents the center hour of the range 906, and the output 908' represents the center hour of the range 908.

The regressor output 902 can have any suitable format that conveys whether the regression determines a meal is occurring, or did occur, at the specified time(s). In some implementations, the regressor output 902 is integer based. For example, the regressor(s) can assign one type of value (e.g., a negative integer) to indicate a determination of no meal, and another type of value (e.g., zero or a positive integer) to indicate a determination of a meal. In some implementations, the regressor output 902 can be issued less often than the individual time points on which the classifier output was based (e.g., the classifier(s) may have generated the classifier output once for every 15-minute period). For example, with every hour the regressor(s) can issue one output (e.g., a single integer) that indicates at what time during that hour the meal is determined to have begun. In some implementations, different (positive) integer values can code respective shorter time periods. For example, the integer zero can indicate the first quarter of an hour, the integer one can indicate the second quarter, and so on.

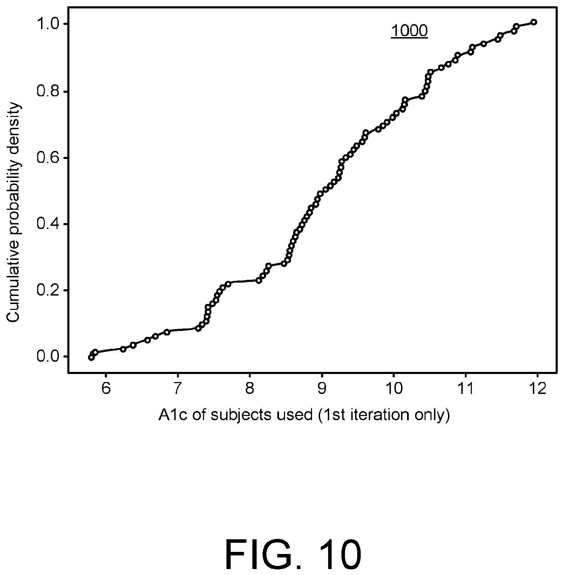

FIG. 10 shows an example of training data 1000 reflecting distribution of a blood protein within a population. The training data can be generated from simulated data and/or from data obtained from one or more actual persons with diabetes. In some implementations, the distribution can be indicated in terms of a cumulative probability density measured against a vertical axis, and feature values measure against a horizontal axis. The training data 1000 can be used with one or more other features described herein. The training data 1000 can be used with one or more systems or devices described elsewhere herein. Any feature can be indicated similarly to the training data 1000. Here, the training data 1000 indicates the probability density for different values of the blood protein A1C.

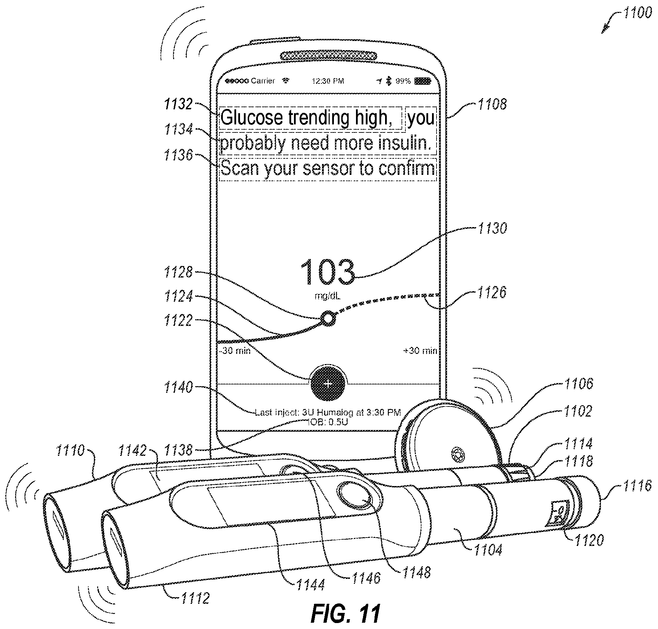

FIG. 11 shows an example of a system 1100 for diabetes management. The system 1100 can be used in combination with, or can include aspects of, one or more other examples described elsewhere herein. Here, the system 1100 includes an insulin injection pen 1102. In some implementations, the insulin injection pen 1102 is considered an insulin injection pen for a certain type of insulin, such as a rapid-acting insulin pen. For example, the insulin injection pen 1102 is a Humalog^{™} pen, a Novolog^{™} pen, or an Apidra^{™} pen. Here, the system 1100 includes an insulin injection pen 1104. In some implementations, the insulin injection pen 1104 is considered an insulin injection pen for a certain other type of insulin having an active time that is longer than an active time for the certain type of insulin, such as a long-acting insulin pen. For example, the insulin injection pen 1104 is a Lantus^{™} pen, a Levemir^{™} pen, a Toujeo^{™} pen, or a Tresiba^{™} pen. Here, the system 1100 includes a glucose monitor 1106 (e.g., a CGM) or another glucose sensor, and a remote user interface device 1108. As shown, each insulin injection pen 1102 and 1104 includes a corresponding pen cap 1110 and 1112, respectively, which are in wireless communication with other components of the system 1100. As shown, the insulin injection pens 1102 and 1104 can include dials 1114 and 1116, respectively, for a user to set a dosage to be delivered, and a respective dose indicator window 1118 and 1120. In some implementations, one or more of the insulin injection pens 1102 and/or 1104 may include dose-capture technology and/or be in wireless communication with other components of the system 1100. Additional details about possible insulin pens and/or insulin pen caps are exemplified below.

Glucose monitor 1106 or another glucose sensor can include any suitable sensor device and/or monitoring system capable of providing data that can be used to estimate one or more blood glucose values. As shown, glucose monitor 1106 or another glucose sensor can be a sensor configured to transmit blood glucose data wirelessly. For example, the glucose monitor 1106 or another glucose sensor can include an optical communication device, an infrared communication device, a wireless communication device (such as an antenna), and/or chipset (such as a Bluetooth device (e.g., Bluetooth Low Energy (BLE), Classic Bluetooth, etc.), an NFC device, an 802.6 device (e.g., Metropolitan Area Network (MAN), a Zigbee device, etc.), a WiFi device, a WiMax device, a Long Term Evolution (LTE) device, cellular communication facilities, etc.), and/or the like. The glucose monitor 1106 or another glucose sensor may exchange data with a network and/or any other devices or systems described in the present disclosure. In some cases, the glucose monitor 1106 or another glucose sensor can be interrogated with an NFC device by the user moving one or more components of the system 1100 near the glucose monitor 1106 or another glucose sensor to power the glucose monitor 1106 or another glucose sensor, and/or to transmit blood glucose data from the glucose monitor 1106 or another glucose sensor, to other components of system 1100. For example, the pen cap 1110 and/or 1112 can exchange data with (e.g., obtain glucose values from) the glucose monitor 1106 or another glucose sensor by being brought in close proximity thereof.

As shown, remote user interface device 1108 is a smartphone in some examples. In some implementations, any suitable remote user interface device can be used, including, but not limited to, a computer tablet, a smartphone, a wearable computing device, a smartwatch, a fitness tracker, a laptop computer, a desktop computer, a smart insulin pen (e.g., the pen caps 1110 and/or 1112), and/or other appropriate computing devices. As shown in the exemplary user interface of the exemplary mobile app running on the depicted smartphone, the user interface can include a bolus calculator button 1122 and optionally other buttons for the user to enter data or to request recommendations. The exemplary user interface can also or instead include a display of blood glucose data (e.g., past, present, and/or predicted). As shown, the user interface includes a graph 1124 of historical data (e.g., from the previous 30 minutes), a continuation 1126 of that graph having projected data, a point indicator 1128 showing the current (or most recent) estimated blood glucose value, and a display 1130 of the current (or most recent) estimated blood glucose value. The user interface can also or instead include text 1132 explaining the glucose data, text 1134 and/or text 1136 providing suggested actions. For example, text 1134 can provide insulin, carbohydrates, or other therapy suggestions. For example, text 1136 can suggest that the user obtain blood glucose data. In some cases, the user interface can permit the user to click on the glucose data or otherwise navigate in the mobile app to obtain more detailed or more complete blood glucose data. In some implementations, the user interface can also or instead provide one or more insights for the benefit of the person with diabetes. For example, an insight can communicate a message along the lines of "You normally have a meal bolus between noon and 2 PM, but didn't bolus today and your blood glucose levels are rising. Did you forget to bolus?"

The user interface can depict insulin data. In some cases, the user interface can indicate an amount 1138 of IOB, which may be only for a particular type of insulin (including, but not limited to, rapid-acting insulin). In some implementations, IOB (sometimes referred to as active insulin) can be defined as the amount of insulin that has been delivered and is still active in the person's body based on estimated duration of insulin action. In some cases, an IOB calculation may be for both rapid-acting and long-acting insulin. In some cases, the user interface can display information 1140, including, but not limited to, the time and/or amounts of the most recent doses of rapid-acting and/or long-acting insulins. In some cases, the user interface can permit the user to click on the insulin data or otherwise navigate in the mobile app to more detailed and/or more complete insulin delivery data. In some cases, a user interface can overlay blood glucose data and insulin delivery data in any suitable format, such as a graphical display of the timing of blood glucose data vs. the timing of insulin delivery data.

In use, a user (e.g., the person with diabetes and/or a caregiver) can use the system 1100 to get recommendations regarding an appropriate insulin dosage. In the case of an upcoming need to deliver long-acting insulin, the text 1134 may be changed to provide a recommended long-acting insulin dosage. In some cases, a recommended dosage may appear on pen cap 1110 and/or 1112. In the case of the user wanting to deliver a bolus of rapid-acting insulin, the user may press bolus calculator button 1122 to enter into a bolus calculator. Any suitable bolus calculator could be used in systems, methods, and devices provided herein. For example, the bolus calculator can provide a user interface for a user to enter a meal announcement as either a correction only, a small meal, a normal sized meal, or a large meal. Upon selecting the meal size, the user interface can provide a recommended bolus dosage based on a number of carbohydrates associated with the corresponding button and optionally based upon blood glucose data. Additionally, or alternatively, dose capture pen caps for the insulin injection pen 1102 and/or 1104 can include a user interface that permits the user to obtain a meal bolus recommendation for different types of meals (small, medium, large; breakfast, lunch, dinner; 10 grams carbs, 20 grams carbs, 45 grams carbs; etc.) and/or announce a meal size, including, but not limited to, a small meal, medium meal, or large meal. In some implementations, the text 1132, 1134, and/or 1136 is presented elsewhere than on the user interface device 1108. For example, presentation can be done on the pen cap 1110 and/or 1112.

The pen cap 1110 and/or 1112 can include at least one display. In some implementations, the pen cap 1110 includes a display 1142. In some implementations, the pen cap 1112 includes a display 1144. The display 1142 and/or 1144 can comprise any suitable type of display technology, including, but not limited to, a dynamic electronic display (e.g., a light-emitting diode display) or a static electronic display (e.g., an E-Ink display). The display 1142 and/or 1144 can present information to a user, including, but not limited to, any information output that is described elsewhere herein. For example, an insulin dosage suggestion and/or an alert or other communication can be presented.

The pen cap 1110 and/or 1112 can include at least one input control. In some implementations, the pen cap 1110 includes a button 1146. In some implementations, the pen cap 1112 includes a button 1148. The button 1146 and/or 1148 can comprise any suitable type of input technology, including, but not limited to, an electronic switch. The button 1146 and/or 1148 can trigger the corresponding pen cap 1110 and/or 1112 to perform one or more operations, including, but not limited to, any operation that is described elsewhere herein.

The pen cap 1110 and/or 1112 can record and/or convey one or more types of pen capping information. Pen capping information (e.g., information about when the pen cap is secured to and/or released from the injection pen) can include information about a current capping period (e.g., the time since the last capping), information about a duration of one or more uncappings (which may also be referred to herein as "decapping(s)"), and/or the timing (e.g., time-of-day or time elapsed since) of each uncapping and each capping. For example, capping information can include data reflecting when the pen cap was placed onto the insulin injection pen, data reflecting when the pen cap was removed from the insulin injection pen, or both. In some embodiments, pen capping information may be presented to a user on a display of the pen cap. In some embodiments, pen capping information may be presented by a speaker in the pen cap. For example, in some embodiments, a pen cap may provide a timer clock that counts up from the last time the pen cap was secured to the injection pen. In some embodiments, a pen cap accessory can wirelessly communicate pen capping information to a remote computing device (e.g., the user interface device 1108 and/or a smartphone, tablet, etc.). In some embodiments, one or more accessories or smart delivery devices can detect other events associated with medication delivery actions and use that information in ways that pen capping information is described herein. For example, in some cases an injection pen accessory may be secured to an injection pen such that it can detect the mechanical movement of the dosing mechanism to determine a time of a dose of medication (and optionally but not necessarily an amount of medication delivered at that time).

Pen capping information may be stored, displayed, and/or analyzed in combination with glucose data to determine user behaviors, such as, for example, whether the person is appropriately dosing insulin for meals and/or to correct elevated blood glucose levels. In some embodiments, pen capping information may be presented on a graphical representation of blood glucose data for the user and presented to a user and/or to a healthcare professional. In some embodiments, blood glucose data from a period of time after each capping event may be evaluated to determine whether the user appropriately dosed insulin for that capping event, e.g., appropriate dose, under dose, or over dose.

In some embodiments, a pen capping event may be disregarded where other information indicates that a dose was not provided. For example, where no change in the dosage selection of the insulin pen (e.g., a dial) was detected, the event may be disregarded. In some embodiments, a pen uncapping and recapping event may be disregarded if the total uncapping time is less than a first threshold (e.g., 4-6 seconds). For example, the threshold may be determined by setting it at an amount of time too short to permit for an injection, but long enough to allow a user to check the end of the pen to see if there is insulin remaining or if there is a needle attached to the pen. In some cases, the total decapping time (the time between an uncapping event and the subsequent recapping) for a decapping event may be analyzed in combination with blood glucose data to determine if there was an injection during that decapping event. In some cases, if the total decapping time exceeds a second threshold period of time (e.g., at least 15 minutes, at least 30 minutes, etc.), blood glucose data and the methods described herein for detecting a meal timing may be used to determine an approximate time of an injection.

Some or all components of the system 1100 can perform one or more operations or functions described elsewhere herein. In some implementations, the system 1100 can determine at least one missed bolus event and associated the event with a meal ingested by the person with diabetes. The system 1100 can issue an alert based on detecting a missed bolus. The alert can be issued substantially in real time (e.g., by allowing for about one hour after the detection of a beginning of a meal before determining that a bolus is missed). The system 1100 can determine insulin use by detecting removal and/or replacement of the pen cap 1110 and/or 1112. The system 1100 can detect an amount of insulin that is injected. In some implementations, the system 1100, or a part thereof, can be an insulin treatment device configured for classifying each of multiple time periods within the period of time regarding whether the time period is associated with ingestion of a meal by the person, and for performing regression analysis on the classified multiple time periods to identify a time period of the classified multiple time periods as corresponding to a beginning of the meal. The system 1100 can associate a missed bolus event with the meal as applicable.

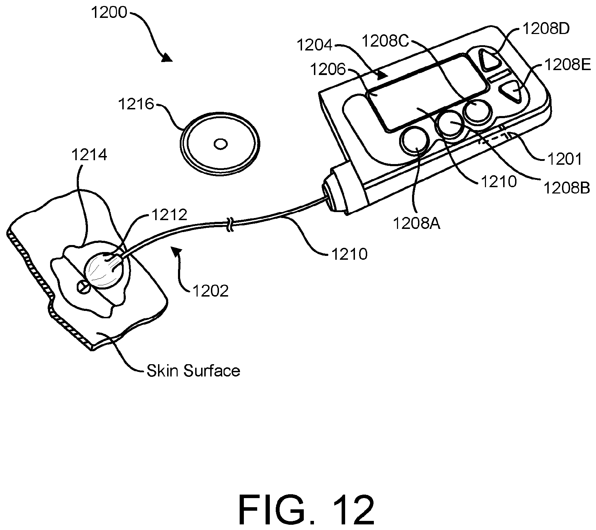

FIG. 12 is a perspective view of an example of an infusion pump system 1200. The infusion pump system 1200 can be portable and have a plunger engagement device 1201 to serve as a reusable pump apparatus (rather than a disposable pump device). In such circumstances, the infusion pump system 1200 may comprise a reusable device that houses the control circuitry and the pump drive system within a single housing construct. In some implementations, the infusion pump system 1200 can include a reusable pump device that houses both controller circuitry and a pump drive system. The infusion pump system 1200 can include a housing that defines a cavity in which a fluid cartridge can be received (not shown for simplicity). For example, the infusion pump system 1200 can be adapted to receive a fluid cartridge in the form of a carpule that is preloaded with insulin or another medicine. The pump drive system can act upon the fluid cartridge to controllably dispense medicine through an infusion set 1202 and into the user's tissue or vasculature. In this embodiment, the user can wear the infusion pump system 1200 on the user's skin under clothing or in the user's pocket while receiving the medicine dispensed through the infusion set 1202. Any of multiple possible mechanisms for delivering medicament (e.g., insulin) can be used. In some implementations, the plunger engagement device 1201 of the infusion pump system 1200 may include a retention spring (not shown) that once activated biases the plunger of the fluid cartridge towards a piston rod of the drive system to increase dosage accuracy.

A user interface 1204 of the infusion pump system 1200 includes a display device 1206 and one or more user-selectable buttons 1208A-1208E. The display device 1206 can include an active area in which numerals, text, symbols, images, or a combination thereof can be displayed. The display device 1206 can be used to communicate a number of settings or menu options for the infusion pump system 1200. For example, the display device 1206 can be used to communicate medicinal delivery information, such as the basal delivery rate, a bolus dosage, a historical record of medicine delivered, the amount of medicine remaining in the fluid cartridge, a communication, an alert, or the like. In another example, the display device 1206 can be used to communicate time and date information, which can be used by the user to determine dosage schedules, bolus delivery times, meal times, or the like.

Accordingly, the user may press one or more of the buttons 1208A-E to shuffle through a number of menus or program screens that show particular settings and data (e.g., review data that shows the medicine dispensing rate, the total amount of medicine dispensed in a given time period, the amount of medicine scheduled to be dispensed at a particular time or date, the approximate amount of medicine remaining in the fluid cartridge, or the like). Also, the user can adjust the settings or otherwise program the infusion pump system 1200 by pressing one or more buttons 1208A-E of the user interface 1204. Thus, the user can contemporaneously monitor the operation of the infusion pump system 1200 from the same user interface 1204.

The infusion set 1202 can include a flexible tube 1210 that extends from the pump device to a subcutaneous cannula 1212 that may be retained by a skin adhesive patch 1214 that secures the subcutaneous cannula 1212 to the infusion site. The skin adhesive patch 1214 can retain the infusion cannula 1212 in fluid communication with the tissue or vasculature of the user so that the medicine dispensed through the tube 1210 passes through the cannula 1212 and into the user's body. In some implementations, the infusion set 1200 can include a glucose sensor 1216 that can indicate one or more EGVs. The glucose sensor 1216 is here illustrated as a component separate from the infusion set 1202 and the infusion pump for clarity (e.g., the glucose sensor 1216 can be removably attached to the skin of the person with diabetes). In some implementations, the glucose sensor 1216 can be an integrated component. The glucose sensor 1216 can include a CGM and/or a BGM, to name just two examples.

Some or all components of the infusion pump system 1200 can perform one or more operations or functions described elsewhere herein. In some implementations, the infusion pump system 1200 can determine at least one missed bolus event and associated the event with a meal ingested by the person with diabetes. The infusion pump system 1200 can issue an alert based on detecting a missed bolus. For example, the alert can be issued to a person with diabetes who uses an insulin pump and can communicate a message along the lines of "You typically bolus X units for lunch and today you bolused Y units (or you did not bolus), do you want to increase the bolused amount to X units?" The alert can be issued substantially in real time (e.g., by allowing for about one hour after the detection of a beginning of a meal before determining that a bolus is missed). The infusion pump system 1200 can determine insulin use by detecting that insulin is being injected into the person with diabetes. The infusion pump system 1200 can detect an amount of insulin that is injected via the infusion pump system 1200. In some implementations, the infusion pump system 1200, or a part thereof, can be an insulin treatment device configured for classifying each of multiple time periods within the period of time regarding whether the time period is associated with ingestion of a meal by the person, and for performing regression analysis on the classified multiple time periods to identify a time period of the classified multiple time periods as corresponding to a beginning of the meal. The infusion pump system 1200 can associate a missed bolus event with the meal as applicable.

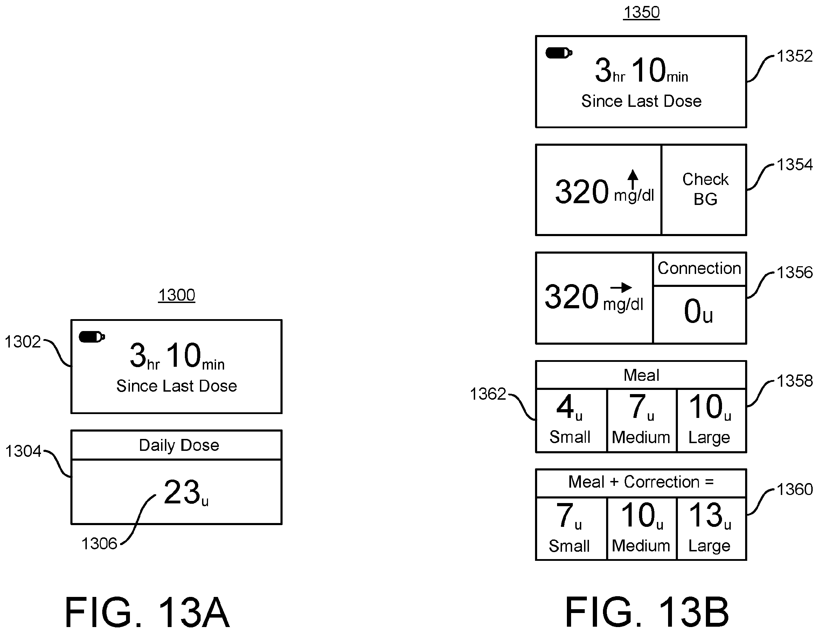

FIGS. 13A-B show examples of graphical user interfaces 1300 and 1350, respectively. The graphical user interfaces 1300 and/or 1350 can be used in combination with one or more other examples described elsewhere herein. Each of the graphical user interfaces 1300 and 1350 can be presented on a suitable display. In some implementations, the graphical user interface 1300 relates to an insulin injection pen for a certain type of insulin, such as a long-acting insulin pen. For example, the graphical user interface 1300 can be presented on the display 1142 and/or 1144 (FIG. 11), and/or on the display device 1206 (FIG. 12). In some implementations, the graphical user interface 1350 relates to an insulin injection pen for a certain other type of insulin, such as a rapid-acting insulin pen. For example, the graphical user interface 1300 and/or 1350 can be presented on the display 1142 and/or 1144 (FIG. 11), and/or on the display device 1206 (FIG. 12).

One or more of the graphical user interfaces 1300 and 1350 can support one or more types of processes relating to insulin therapy management. In some implementations, a pairing process can be supported that seeks to establish a connection between the pen cap and one or more other devices. For example, connection between the pen cap and a smartphone (e.g., the remote user interface device 1108 in FIG. 11) can be established. In some implementations, a daily use process can be supported that corresponds to the person's with diabetes ordinary use of the pen cap, after one or more successful pairings with the other device(s), to manage diabetes therapy.

In some implementations, a daily use workflow for the graphical user interface 1300 can include a default screen (e.g., presenting a name of the insulin currently contained in the insulin injection pen), a timer screen 1302, and/or a dose suggestion screen 1304. For example, the person with diabetes can cycle through two or more screens using an input control (e.g., the button 1146 and/or 1148 in FIG. 11, and/or one or more of the buttons 1208A-E in FIG. 12). For example, the graphical user interface 1300 can automatically return to presenting the default screen if no action is undertaken using the pen cap (e.g., by way of a button other otherwise) within a predefined time period.

In some implementations, the timer screen 1302 presents a time that has elapsed since the last dose (e.g., since a most recent capping/uncapping event that qualifies as an indicator of insulin dosage). For example, the timer screen currently states that it has been 3 hours and 10 minutes since the last dose. In some implementations, the elapsed time can be based on actual dosage timing (e.g., as reported by an injection device), or on a proxy for insulin injection timing other than capping/uncapping.

In some implementations, the dose suggestion screen 1304 presents an insulin dosage suggestion 1306. The insulin dosage suggestion 1306 can contain one or more recommendations to the person with diabetes. For example, the insulin dosage suggestion 1306 currently suggests that the person with diabetes should ingest a daily dose of insulin (e.g., long-acting insulin) corresponding to an amount of 23u (units).

In some implementations, a daily use workflow for the graphical user interface 1350 can include a default screen (e.g., presenting a name of the insulin currently contained in the insulin injection pen), a timer screen 1352, a blood glucose value screen 1354, a blood glucose value screen 1356, a dose suggestion screen 1358, and/or a dose suggestion screen 1360. For example, the person with diabetes can cycle through two or more screens using an input control (e.g., the button 1146 and/or 1148 in FIG. 11, and/or one or more of the buttons 1208A-E in FIG. 12). For example, the graphical user interface 1350 can automatically return to presenting the default screen if no action is undertaken using the pen cap (e.g., by way of a button other otherwise) within a predefined time period.

In some implementations, the timer screen 1352 presents a time that has elapsed since the last dose (e.g., since a most recent capping/uncapping event that qualifies as an indicator of insulin dosage). For example, the timer screen currently states that it has been 3 hours and 10 minutes since the last dose. In some implementations, the elapsed time can be based on actual dosage timing (e.g., as reported by an injection device), or on a proxy for insulin injection timing other than capping/uncapping.

In some implementations, the blood glucose value screens 1354 and/or 1356 presents data obtained by way of a glucose monitor or another glucose sensor (e.g., a CGM). For example, the blood glucose value screen 1354 can present a value of a blood glucose estimate (e.g., 320 mg/dL), present a trend indication (e.g., an arrow pointing up, or towards the right and up, or to the right, or towards the right and down, or down), and/or a suggestion that the person with diabetes should check his or her blood glucose (BG). For example, the blood glucose value screen 1356 can present a value of a blood glucose estimate (e.g., 320 mg/dL), present a trend indication (e.g., an arrow pointing up, or towards the right and up, or to the right, or towards the right and down, or down), and an insulin dosage suggestion as to whether the person with diabetes should ingest a correction dose (e.g., here zero units is recommended).

In some implementations, the dose suggestion screen 1358 presents an insulin dosage suggestion 1362 relating to a meal. The insulin dosage suggestion 1362 can contain one or more recommendations to the person with diabetes based on information about the meal. For example, the insulin dosage suggestion 1362 currently suggests that the person with diabetes should ingest 4 units of insulin if the meal is a small meal, 7 units if the meal is a medium meal, and 10 units if the meal is a large meal. In some implementations, the pen cap for which the graphical user interface 1350 is implemented may work in combination with one or more glucose monitors or another glucose sensor. For example, the graphical user interface 1350 can work with a CGM. As another example, the graphical user interface 1350 can work with a BGM.

The graphical user interface 1300 and/or 1350 can present one or more other types of information not illustrated in the present examples. Such information can include, but is not limited to, alerts (e.g., regarding a missed bolus), a communication (e.g., regarding a late bolus), messages regarding a need to charge the pen cap, messages that the pen cap is currently synchronizing data, and/or error messages (e.g., urging the person with diabetes to consult the remote user interface device 1108 (FIG. 11).

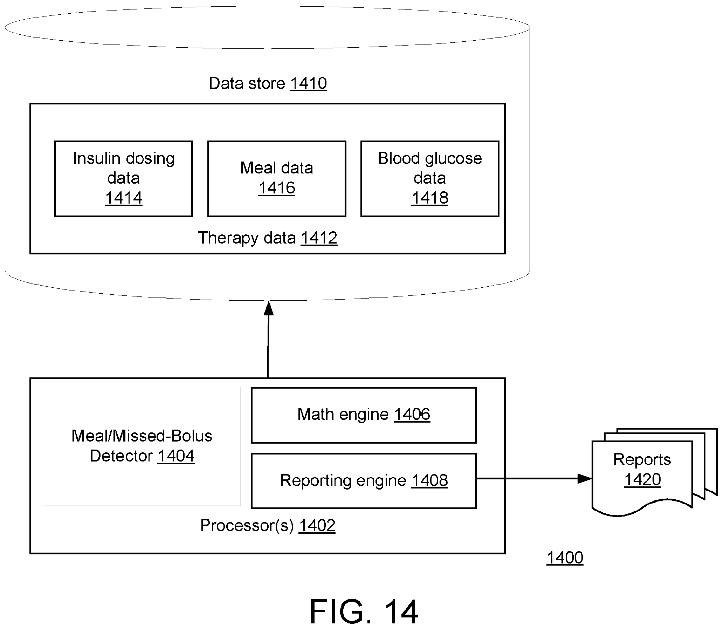

FIG. 14 shows a computing platform 1400 for insulin-based management of diabetes. The computing platform 1400 can be used with one or more other examples described elsewhere herein. In disclosed embodiments, computing platform 1400 is, or is operative to be executed as, a data processing system, and more specifically, as a data processing system for detecting a meal and/or a missed bolus for a person with diabetes. The computing platform 1400, or one or more components thereof, can be implemented using one or more components exemplified below in the description of FIG. 20.

Computing platform 1400 may include data store 1410 and processor(s) 1402. Data store 1410 may include therapy data 1412. Therapy data 1412 is data related to insulin therapy of one or more persons. Therapy data 1412 can include insulin dosing data 1414, meal data 1416, and blood glucose data 1418. For example, therapy data 1412 can include blood glucose values and/or bolus information relating to a period of time. Alternatively, or additionally, therapy data 1412 may include exercise data, sleep data, and/or physiological parameters of a patient (e.g., ISF or CR).

Blood glucose data 1418 may include data about blood glucose in a human body at one or more times. Blood glucose data 1418 may include measurements of blood glucose levels, for example, raw blood glucose measurements, blood glucose estimates based on blood glucose measurements, and/or aggregations of the same (e.g., averages, trends and metrics). Blood glucose data 1418 may include date and time (e.g., a timestamp), and a value for each blood glucose measurement. In disclosed embodiments, any suitable glucose sensor may provide blood glucose data 1418, for example, a CGM, a flash glucose monitor, a BGM. In the case of CGMs and flash glucose monitors, they may be configured to provide blood glucose data 1418 based on interstitial fluid glucose levels of a person, which may be correlated to blood glucose levels. A BGM may be configured to provide blood glucose data based on a blood sample. Accordingly, the term "blood glucose" is not limited herein to using just blood glucose data, values, levels etc., but is also intended to include interstitial fluid glucose levels, intermediate measurements, and equivalents thereof.

Insulin dosing data 1414 may include dosing event data. Dosing event data may include data about insulin dosing actions at one or more times and may include, for example, a dosing time or time range, type of insulin (e.g., long-acting (LA) insulin and rapid-acting (RA) insulin) dosed, brand of insulin, and/or amount of dosed insulin. In some embodiments, dosing event data may include an indication of a dosing mechanism, for example, injection pen, inhaler, or infusion pump. In some embodiments, dosing event data may include an indication of whether dosing event data, in part or in whole, is based on an actual dosing action (e.g., detecting insulin delivery, for example, based on a manual action of a pump or a control signal configured to cause insulin delivery), user tracking of dosing actions (e.g., a person with diabetes or caregiver enters a dose using a therapy application executing on a mobile device), or inferred dosing actions (e.g., from capping/uncapping of an injection pen).

Processor(s) 1402 may be configured to execute a number of engines for performing disclosed embodiments. Processor(s) 1402 can include a meal/missed-bolus detector 1404, math engine 1406, and reporting engine 1408.

Meal/missed-bolus detector 1404 may be configured, generally, to process therapy data 1412 or part(s) of therapy data 1412, and detect a meal and/or a missed bolus. A part of therapy data 1412 processed by the meal/missed-bolus detector 1404 may correspond to a particular time period. In one embodiment, meal/missed-bolus detector 1404 may return predictions of a discrete variable, such as to detect whether the person with diabetes ingests a meal and/or misses a bolus. The meal/missed-bolus detector 1404 may output one or more discrete variables, or can cause one or more other actions to be taken. The meal/missed-bolus detector 1404 may include one or more classifiers and one or more regressors, and can be trained using one or more supervised and/or unsupervised learning techniques, including those described in more detail in this disclosure.

Math engine 1406 may be configured to perform various statistical calculations using therapy data 1412 and results provided by meal/missed-bolus detector 1404. In various embodiments, statistical calculations may include, for example, frequency calculations, confidence calculations, probability calculations, and more.

Reporting engine 1408 may be configured, generally, to generate one or more reports 1420 responsive to meal/missed-bolus detector 1404 and/or math engine 1406. Reports 1420 may include descriptions of retrospective studies performed at computing platform 1400, and/or determinations resulting therefrom, and may include, for example, patient identifiers, descriptions of retrospective time periods, assigned class labels, the class labels and more.

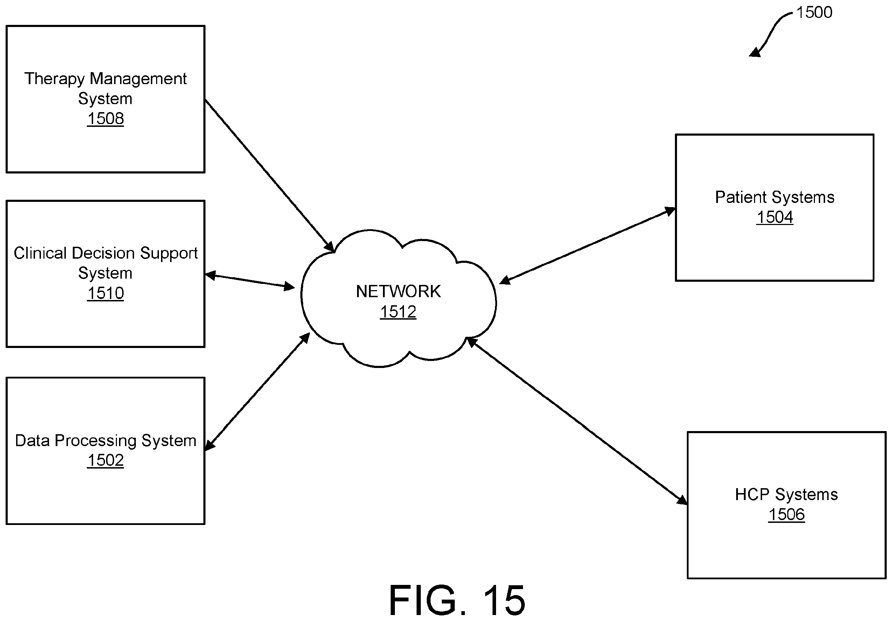

FIG. 15 shows a simplified block diagram of an example of a system for insulin-based management of diabetes. Any suitable technique used by one of ordinary skill in the art in the field of data science to calculate and/or express probabilities may be used with disclosed embodiments. Some embodiments relate, generally, to insulin therapy systems and elements thereof that incorporate systems, methods and devices for detecting a meal and/or a missed bolus. FIG. 15 shows a system 1500 for insulin therapy, in accordance with disclosed embodiments. The system 1500 can be used with one or more other examples described elsewhere herein. The system 1500, or one or more components thereof, can be implemented using one or more components exemplified below in the description of FIG. 20.

In the embodiment shown in FIG. 15, data processing system 1502, clinical decision support system 1510, and therapy management system 1508 are computing platforms configured, generally, to provide various services related to insulin therapy, in whole or in part, to each other and to health care provider (HCP) systems 1506 and patient systems 1504. The data processing system 1502, clinical decision support system 1510, therapy management system 1508, HCP systems 1506, and patient systems 1504 can be connected by at least one network 1512 (e.g., the Internet). HCP systems 1506 may include, for example, portals, dashboards, electronic medical record systems, computing platforms executing the same, and more.

In some embodiments, therapy management system 1508 may be or includes one or more computing platforms configured to receive and store therapy data (such as therapy data 1412 in FIG. 14) and physiological parameters about patients, issue alarms and alerts, and manage therapy settings for insulin delivery systems. In short, the therapy management system 1508 performs insulin-based management of diabetes.

In some embodiments, clinical decision support system 1510 may be one or more computing platform configured as a health data technology system for assisting HCPs with clinical decision making tasks, and more specifically in this example, assist HCPs with clinical decision making tasks related to a person's insulin therapy. In disclosed embodiments, clinical decision support system 1510 is configured to assist with insulin-based management of diabetes, and automatically analyzes therapy data, identifies clinically relevant patterns in a person's therapy from the therapy data, and provides data and recommendations to HCP systems 1506 based on those patterns. A goal of embodiments of clinical decision support system 1510 is to improve outcomes for persons with diabetes by facilitating communication of clinically relevant "insights" about a person's insulin-based therapy to patient systems 1504 and HCP systems 1506 as well as by facilitating communication of therapy related advice from HCP systems 1506 to patient systems 1504. For example, the clinical decision support system 1510 can issue alerts and/or communications regarding missed or late boluses.

In disclosed embodiments, data processing system 1502 may be or include one or more computing platforms configured to process therapy data stored at, or received from, therapy management systems 1508 and/or clinical decision support system 1510. In one embodiment, data processing system 1502 may, among other things, include one or more elements of computing platform 1400 (FIG. 14), including meal/missed-bolus detector 1404. In this manner, data processing system 1502 may be configured to perform meal and/or missed-bolus detection for therapy management system 1508 and/or clinical decision support system 1510.

By way of example, data processing system 1502 may perform meal detection, and/or missed-bolus detection, on therapy data stored at clinical decision support system 1510 and provide one or more reports (e.g., report 1420 in FIG. 14) detailing one or more labeled retrospective time periods, as well as one or more determinations regarding a detected meal and/or a missed bolus. Clinical decision support system 1510 may use the data in the reports to trigger insights and/or recommendations that it sends to HCP systems 1506. Upon HCP system 1506 accessing messages from clinical decision support system 1510, data from the reports may be included in such message or accessible by HCP systems 1506 - e.g., accessible if HCP systems 1506 requests data to support an insight or recommendation described in a message.

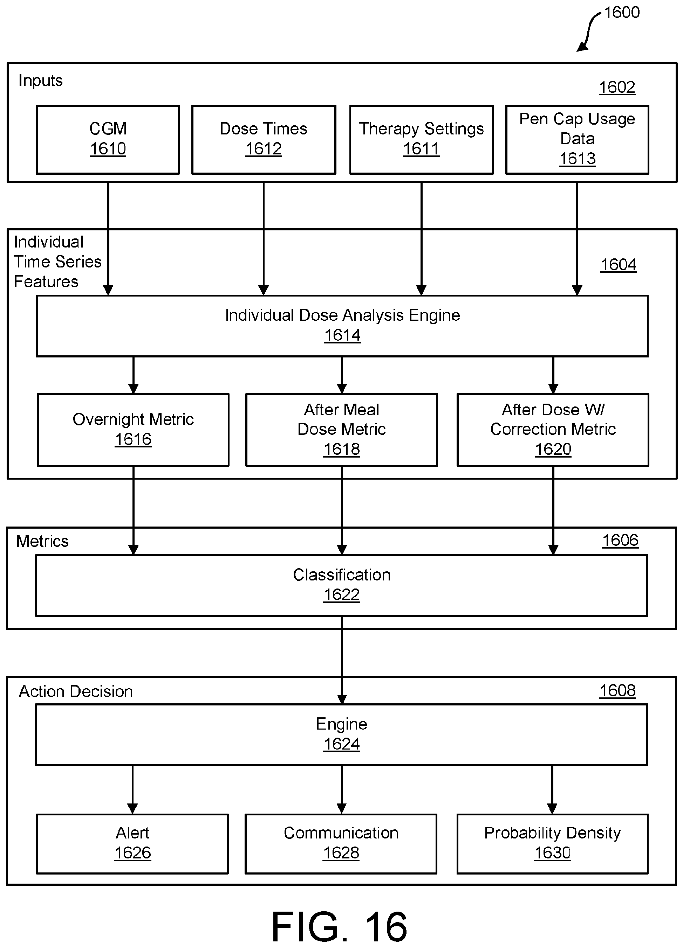

FIG. 16 shows a diagram of an example of a system 1600 for diabetes management. The system 1600 can be used in combination with, or can include aspects of, one or more other examples described elsewhere herein. The system 1600, or one or more components thereof, can be implemented using one or more components exemplified below in the description of FIG. 20.

The system 1600 can include an inputs layer 1602, an individual time series features layer 1604, a metrics layer 1606 for making determinations regarding features, and an action decision layer 1608. The inputs layer 1602 can facilitate that the system 1600 obtains data regarding blood glucose levels and/or dosage times. In some implementations, the inputs layer 1602 facilitates connection with a CGM 1610 (e.g., the glucose monitor 1106 of FIG. 11, or another glucose sensor). In some implementations, the inputs layer 1602 facilitates receipt of therapy settings 1611. For example, the therapy settings 1611 specifies one or more doses of insulin, and/or characteristics of the dose(s) of insulin, for a person with diabetes. In some implementations, the inputs layer 1602 facilitates receipt of dose times data 1612 (e.g., by way of detecting capping data from a pen cap of an insulin injection pen, or using another proxy for dosage times). In some implementations, the inputs layer 1602 facilitates receipt of pen cap usage data 1613. For example, the pen cap usage data 1613 can be based on one or more capping or uncapping event of a pen cap for an insulin injection pen. One or more of the CGM 1610, therapy settings 1611, dose times data 1612, or the pen cap usage data 1613 can be used in some implementations. For example, the dose times data 1612 can be determined or inferred from the pen cap usage data 1613. In some implementations, the inputs layer 1602 can receive about two weeks of blood glucose data and dosage timing information in a batch, or information can be received substantially in real time. In some implementations, the time period between therapy parameter adjustments can be configurable by an algorithm. For example, the time period between consecutive therapy parameter adjustments can be referred to as an iteration of the algorithm. The inputs layer 1602 can provide some or all of the information to the individual features/metrics layer 1604.

The individual time series features layer 1604 can serve to select the relevant data features that will help with the evaluation of whether a meal is ingested and/or a bolus is missed. In some implementations, the individual time series features layer 1604 includes an individual dose analysis engine 1614. For example, the individual dose analysis engine 1614 can identify one or more individual dose times in dose times data 1612, identify one or more blood glucose values from the CGM 1610 corresponding to the identified one or more individual dose times, calculate one or more other metrics such as µ* or σ* from a set of time series features of interest, and/or process the feature(s) to make a determination of whether the person with diabetes ingests a meal and/or misses a bolus.

The individual dose analysis engine 1614 can select one or more metrics to be applied in analyzing blood glucose values and/or bolus information. In some implementations, the individual dose analysis engine 1614 applies one or more of a metric 1616 relating to overnight insulin dosage, a metric 1618 relating to insulin dosage taken after a meal, and/or a metric 1620 relating to an after-meal dose with a correction dose. For example, the metric 1616 can be applied to a portion of the blood glucose data that can correspond to sleeping time for the person with diabetes (e.g., sleeping time can be an example of fasting time for the person with diabetes). For example, the metric 1618 can be applied to a portion of the blood glucose data that corresponds to time in connection with an ingested meal. For example, the metric 1620 can be applied to a portion of the blood glucose data that corresponds to time in connection with an eaten meal, wherein the dose times data 1612 indicates that the person with diabetes took a correction dose (e.g., an insulin dosage suggestion made to the person with diabetes may have included a correction bolus recommendation, and capping data from the pen cap indicates that the correction bolus recommendation was accepted by the person with diabetes.)

In the metrics layer 1606, one or more of the metrics selected by the individual dose analysis engine 1614 can be applied to detect a meal and/or missed bolus. In some implementations, the metrics layer 1606 includes a classification component 1622 that classifies one or more instances of blood glucose data, and/or one or more instances of bolus information, using the selected metric(s). For example, the classification component 1622 can apply the metric(s) to blood glucose values and bolus information to determine whether a person with diabetes ingests a meal or misses a bolus after a meal.

The action decision layer 1608 can make one or more decisions based on the determination by the metrics layer 1606. In some implementations, the action decision layer 1608 includes an engine 1624. For example, the engine 1624 can evaluate classifier outputs generated by the metrics layer 1606 and decide when one or more meals began and/or when the person missed a bolus dose. The action decision layer 1608 can cause one or more alert 1626 to be generated. The action decision layer 1608 can cause one or more communication 1628 to be issued for the person with diabetes. The action decision layer 1608 can cause one or more probability density record 1630 regarding the person with diabetes to be generated.

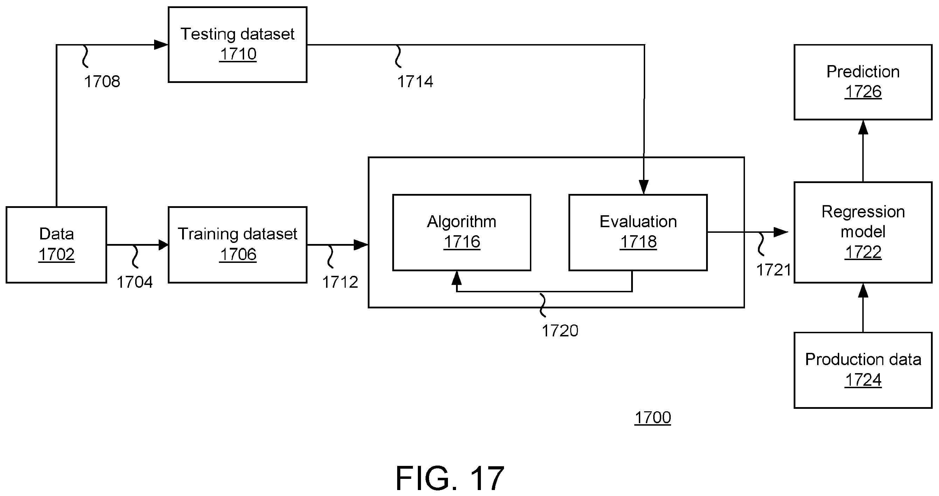

FIG. 17 shows an example of a system 1700 for training a regression model. The system 1700 can be used with one or more other examples described elsewhere herein. The system 1700 can involve the following stages: gathering data 1702; preprocessing the data 1702 at 1704 (into a training dataset 1706) and at 1708 (into a testing dataset 1710); researching the regression model, in terms of an algorithm 1716 and an evaluation 1718 that are based on the training dataset 1706 at 1712, by way of one or more iterations 1720; testing the regression model using the testing dataset 1710 at 1714; training, at 1721, a regression model 1722; and evaluating the regression model 1722 using production data 1724 to make one or more predictions 1726. In some implementations, the training dataset 1706 can include both a set of training data and a set of validation data.

The data 1702 can include actual patient data or simulated data, or combinations thereof. For example, data can be provided on a real-time basis or from storage. The data 1702 can be preprocessed to clean the data 1702. For example, the data 1702 can be converted from a raw format (e.g., a format as collected) into a format suitable for regression analysis. As another example, noise can be removed from the data 1702, and/or inconsistencies in the data 1702 can be removed. As another example, missing values in the data 1702 can be ignored (e.g., by removing a remainder of a column or row of the missing value). The training can involve supervised or unsupervised learning. In some implementations, supervised learning involves regression to learn how to predict a numerical value of a continuous variable.

The regression model 1722 can take as inputs at least blood glucose values for a person with diabetes and bolus information (e.g., time-of-administration data) for the insulin therapy treatment. In some implementations, the blood glucose values are read, estimated, or inferred using one or more devices (e.g., a CGM or a BGM). For example, blood glucose values can be collected from the person with diabetes at regular intervals. In some implementations, the time-of-administration information (e.g., when the person ingested insulin, sometimes referred to as time-of-bolus information) can be read, estimated, or inferred using one or more devices (e.g., an insulin injection pen, or a pen cap of the insulin injection pen). For example, time-of-administration information can be collected from the person with diabetes at regular intervals.

In some implementations, the diabetes therapy management system (e.g., a smartphone, insulin injection pen, or a pen cap) facilitates receipt of dose times data (e.g., by way of detecting capping data from a pen cap of an insulin injection pen, or using another proxy for dosage times). In some implementations, pen cap usage data can be detected. For example, the pen cap usage data can be based on one or more capping or uncapping events of a pen cap for an insulin injection pen. One or more of dose times data or pen cap usage data can be used in some implementations. For example, the dose times data can be determined or inferred from the pen cap usage data. In some implementations, about two weeks of blood glucose data and dosage timing information can be received. In some implementations, the time period between therapy parameter adjustments can be configurable by an algorithm. For example, the time period between consecutive therapy parameter adjustments can be referred to as an iteration of the algorithm.

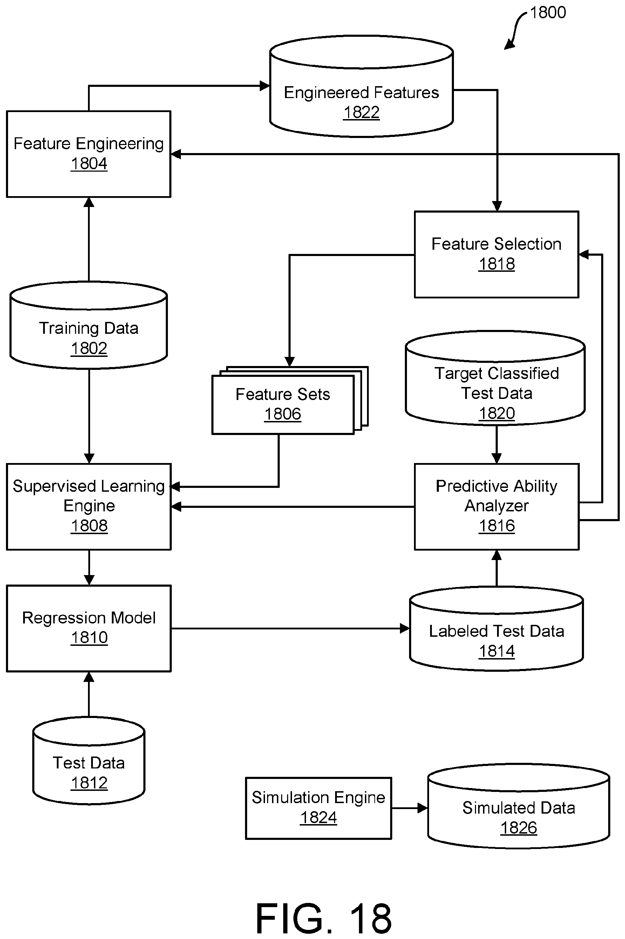

FIG. 18 shows a functional block diagram of system 1800 for training a regression model (such as meal/missed-bolus detector 1404 in FIG. 14) using machine learning techniques, in accordance with disclosed embodiments. The system 1800 can be used with one or more other examples described elsewhere herein.

In a contemplated operation, supervised learning engine 1808 trains regression model 1810 using training data 1802 and sets of engineered features (i.e., feature sets 1806) selected for model training purposes. In some embodiments, regression model 1810 is a function or algorithm that detects meal ingestion and/or a missed bolus. An initial "best guess" may be used for regression model 1810 which is then continually improved by supervised learning engine 1808. In disclosed embodiments, regression model 1810 and supervised learning engine 1808 may implement any suitable supervised learning algorithms and ensemble methods thereof for performing embodiments of the disclosure, including, for example, a Random Forest regressor, a linear support vector regressor (SVR), an SVR with radial basis function (RBF) kernel, Lasso, or Ridge regression model. Disclosed embodiments may also implement supervised learning algorithm(s) that do not use feature selection, including, for example, one class support vector machine (SVM) without feature selection, and logistic regression without feature selection.

In one embodiment, training data 1802 is labeled therapy data associated with one or more persons with diabetes. A person with diabetes may be chosen so they are representative of a desired domain of physiologies, eating behaviors, exercise behaviors, sleeping behaviors, diurnal profile variation, and more.

In disclosed embodiments, feature sets 1806 are sub-sets of features engineered (i.e., formed) in the training data 1802 and used by supervised learning engine 1808 to train any regression model. In one embodiment, feature sets 1806 are created using a feature selection process for selecting a subset of features included in a feature domain created using feature engineering techniques. Features in a feature domain may include, for example, EGV, a ROC of EGV, a ROC of the ROC of EGV, a bolus distance (e.g., a bolus announcement spread out over a finite time), a bolus density (e.g., a bolus probability density used as a proxy for meal propensity), µ* (e.g., a geometric mean of a feature, over a time period or the entire available data span), σ* (e.g., a geometric standard deviation of a feature, over a time period or the entire available data span).

That is, one or more feature sets can be constructed, such as by a selection process at a feature engineering stage. The feature selection process can be performed using the feature sets and the data to obtain a training feature set. Feature sets 1806 may be selected from the feature domain using any suitable feature selection technique or combination of techniques for trying features in the feature domain and identifying important features, including, for example, sequential forward feature selection, sequential backward elimination, and tree-based feature selection algorithms.

Labeled test data 1814 is test data 1812 classified and labeled by regression model 1810 during successive iterations of system 1800. The system 1800 includes target classified test data 1820. Labeled test data 1814 is the "true" or "target" labels for test data 1812. Stated another way, it is the labeling result that is the target for regression model 1810. Predictive ability analyzer 1816 assess the predictive ability of regression model 1810 by comparing the labels of the labeled test data 1814 that were predicted by the regression model 1810 to the true values in the target test data. Any suitable technique for calculating and/or assessing validity of a model may be used by predictive ability analyzer 1816, including for example, precision, recall, number of detected events versus number of true events, confusion matrix, area-under-the-free-curve (AUC), receiver operating characteristics (ROC) curve. Techniques such as Grid search combined with cross validation, and N-fold cross-validation can be used for hyper parameter tuning of a regressor.

Feature selection 1818 receives assessment results from predictive ability analyzer 1816 and, in response, changes feature sets 1806 to attempt to improve accuracy and/or attempt to simplify feature sets 1806. As non-limiting examples, changes to feature sets 1806 may include, changing weighting for features of feature sets 1806, adding features to feature sets 1806 to attempt to improve accuracy of predictions, removing unnecessary features from feature sets 1806, and combinations thereof.

Feature engineering 1804 receives assessment results from predictive ability analyzer 1816 and, in some cases, performs feature engineering techniques to extract new features from test data 1812 and add those features to engineered features 1822. These new features may be used in the feature selection process by feature selection 1818.

In one embodiment, system 1800 may include simulation engine 1824 configured to generate simulation data 1826 from which training data 1802 and test data 1812 may be obtained. Simulation engine 1824 may be configured to simulate insulin therapy scenarios for a variety of persons with diabetes. The profiles can be created so as to represent a cross-section of persons with diabetes in terms of characteristics such as physiology (e.g., age, weight, height, complicating health conditions, diurnal profile variation, etc.), lifestyle (e.g., eating behaviors, exercise behaviors, sleeping behaviors, etc.), socio-economic factors (e.g., income, race, geographic location, marriage status, child status, etc.), differences in how persons measure and track meal intake, and differences in the operation and quality of insulin delivery systems and components the persons use. In one embodiment, simulation engine 1824 is configured to model for missing therapy data due to, for example, lost components, failure to input therapy related data, failure to wear a glucose monitor, and lost Bluetooth connection.

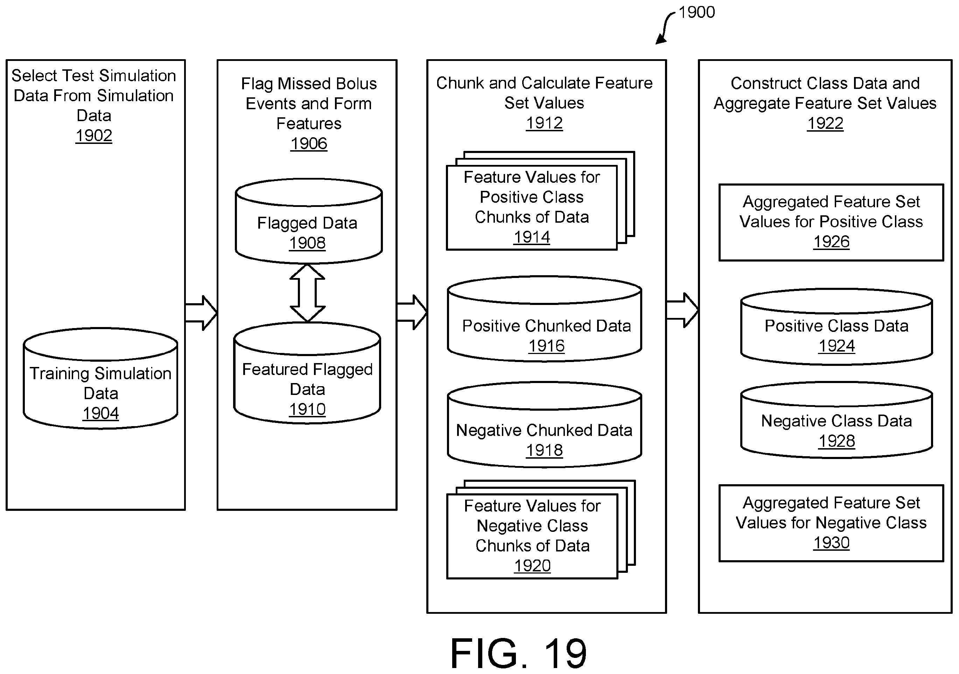

Some disclosed embodiments relate, generally, to obtaining training data and test data, such as training data 1802 and test data 1812, from simulation data such as simulation data 1826. FIG. 19 shows a functional block diagram of an example of a data journey 1900, for creating training data from simulation data, in accordance with disclosed embodiments. Each stage of data journey 1900 is shown as an operational block that describes at least some notable intermediate data elements of data journey 1900.

In operational block 1902, training simulation data 1904 is obtained by selecting part of simulation data (such as simulation data 1826 in FIG. 18)) to be training simulation data 1904. In one embodiment, 90 days' worth of simulated data is obtained and the first 60 days of simulation data is selected to be training simulation data 1904 and the last 30 days of simulation data is selected to be test simulation data.

In operational block 1906, each missed bolus event is flagged in the training simulation data 1904 to obtain flagged data 1908. Notably, each true missed bolus in the simulation data 1904 is known for each person with diabetes that was part of a simulation.

In operational block 1906, feature engineering techniques are used on flagged data 1908 to obtain feature set 1910. More specifically, feature engineering techniques are used to form features in flagged data 1908 to obtain the feature set 1910.

In operational block 1912, feature set 1910 is chunked to obtain positive chunked data 1916 and negative chunked data 1918. In various embodiments, a chunk of data is data that is relevant to a class (here a positive class or negative class), and a chunk of data may itself be formed by aggregating sub-units of data. Positive chunked data 1916 are chunks of data associated with a positive class (i.e., there is a missed bolus event detected). In one embodiment, positive chunked data 1916 may be obtained by aggregating feature set 1910 (here, training data) corresponding to the next 60 minutes' worth of observations after each missed bolus event. Further, a 60 minute chunk of therapy data may be formed of therapy data corresponding to 12 instances of consecutive 5 minute observations.

Negative chunked data 1918 are chunks of data associated with a negative class (i.e., no missed bolus event detected). In one embodiment, negative chunked data 1918 may be obtained by aggregating training data corresponding to a 60 minutes' worth of observations where (1) the observations in the 60 minutes are consecutive in timestamps; (2) chunks of chunked data 1918 do not intersect (i.e., the intersection of chunks of chunked data 1918 is empty); and (3) no chunks of chunked data 1916 and chunks of chunked data 1918 intersect (i.e., the intersection of chunks of chunked data 1916 and chunked data 1918 is empty). In one embodiment, available chunks of data are randomly selected to form chunked data 1916 and/or chunked data 1918. In one embodiment, a number of chunks selected for chunked data 1916 is substantially the same as the number of chunks selected for chunked data 1918.

Feature values 1914 for positive chunked data 1916 and feature values 1920 for negative chunked data 1918 are obtained for chunks of chunked data 1916 and chunks of chunked data 1918, respectively, in operational block 1912. In various embodiments, feature values may be calculated for a chunk of data or one or more smaller observational units of a chunk of data. For example, feature values may be calculated using therapy data for each of the 5 minute observational units that form a 60 minute chunk of therapy data.

In operational block 1922, positive class data 1924 and negative class data 1928 are obtained, and more specifically, are constructed from chunks of chunked data 1916 and chunks of chunked data 1918, respectively. In one embodiment, each positive class data 1924 is formed by labeling the constituent chunks of data of chunked data 1916 with a positive class identifier (e.g., "true" or "1") and copying the labeled chunks of data into positive class data 1924. Similarly, in one embodiment, each negative class data 1928 is formed by labeling the constituent chunks of data of chunked data 1918 with a negative class identifier (e.g., "false" or "0") and copying the labeled chunks of data to negative class data 1928.

In operational block 1922, aggregated feature set values 1926 for positive class data 1924 and aggregated feature set values 1930 for negative class data 1928 are obtained. In one embodiment, aggregated feature set values 1926 for positive class data 1924 and aggregated feature set values 1930 for negative class data 1928 are formed by aggregating feature values 1914 for positive chunked data 1916 and feature values 1920 for negative chunked data 1918, respectively, on a feature by feature basis into a single value for the chunk. In various embodiments, any suitable statistical method for aggregating may be used, including, for example, one or more of mean, median, a commercially available aggregate function (e.g., first value). In various embodiments, training data, such as training data 1802 (FIG. 18), may be obtained by combining positive class data 1924 and negative class data 1928.

In one embodiment, training data obtained as a result of data journey 1900 may be characterized as balanced training data, i.e., having a substantially equal number of observations from both classes (e.g., "detected missed bolus" and "no detected missed bolus"). Since the probability of a missed bolus event is lower than the probability of no missed-bolus event, the observations in simulation data 1826 (FIG. 18) should (in theory) be imbalanced, more specifically, the negative observations should outweigh the positive observations. So, when obtaining balanced training data, a majority of negative observations may not be considered, and so there is potential for data loss. In one embodiment, any impact of data loss is alleviated by using ensemble or bagging techniques.

In disclosed embodiments, test data, such as test data 1812 (FIG. 18), may be obtained in a manner similar to data journey 1900 described above, except that the entire set of test simulation data may be chunked. Test data may be imbalanced (i.e., does not have to be balanced). So, in one embodiment, chunks may be formed by applying a rolling window to test simulation data. For example, 60 minute chunks may be formed by travelling across an entire test simulation data in 5 minute by 5 minute observational units, person by person. Features may be formed for each rolling window by aggregating values using suitable statistical methods similar to training data as described above.

In some cases, there may not be enough consecutive observational sub-units available to form a full chunk of data. Using the example of a 60 minute chunk of data formed of 5-minute observational units, after a missed bolus event there may be 6 of the 5-minute observational units and 5 discontinuities (e.g., gaps between observations). So, in one embodiment, a chunk of data is discarded if fewer than a predetermined number of consecutive observational units is available. Each chunk may be assigned a label with an appropriate class identifier to indicate that it is associated with a missed bolus or no missed bolus. In one embodiment, a chunk of data may be assigned a positive class label if any missed bolus event is within plus-minus 5 minutes of a chunk start time, and assigned a negative class label if otherwise.

FIG. 20 illustrates an example architecture of a computing device 2000 that can be used to implement aspects of the present disclosure, including any of the systems, apparatuses, and/or techniques described herein, or any other systems, apparatuses, and/or techniques that may be utilized in the various possible embodiments.

The computing device illustrated in FIG. 20 can be used to execute the operating system, application programs, and/or software modules (including the software engines) described herein.

The computing device 2000 includes, in some embodiments, at least one processing device 2002 (e.g., a processor), such as a central processing unit (CPU). A variety of processing devices are available from a variety of manufacturers, for example, Intel or Advanced Micro Devices. In this example, the computing device 2000 also includes a system memory 2004, and a system bus 2006 that couples various system components including the system memory 2004 to the processing device 2002. The system bus 2006 is one of any number of types of bus structures that can be used, including, but not limited to, a memory bus, or memory controller; a peripheral bus; and a local bus using any of a variety of bus architectures.

Examples of computing devices that can be implemented using the computing device 2000 include a desktop computer, a laptop computer, a tablet computer, a mobile computing device (such as a smart phone, a touchpad mobile digital device, or other mobile devices), or other devices configured to process digital instructions.

The system memory 2004 includes read only memory 2008 and random access memory 2010. A basic input/output system 2012 containing the basic routines that act to transfer information within computing device 2000, such as during start up, can be stored in the read only memory 2008.

The computing device 2000 also includes a secondary storage device 2014 in some embodiments, such as a hard disk drive, for storing digital data. The secondary storage device 2014 is connected to the system bus 2006 by a secondary storage interface 2016. The secondary storage device 2014 and its associated computer readable media provide nonvolatile and non-transitory storage of computer readable instructions (including application programs and program modules), data structures, and other data for the computing device 2000.

Although the example environment described herein employs a hard disk drive as a secondary storage device, other types of computer readable storage media are used in other embodiments. Examples of these other types of computer readable storage media include magnetic cassettes, flash memory cards, digital video disks, Bernoulli cartridges, compact disc read only memories, digital versatile disk read only memories, random access memories, or read only memories. Some embodiments include non-transitory media. For example, a computer program product can be tangibly embodied in a non-transitory storage medium. Additionally, such computer readable storage media can include local storage or cloud-based storage.

A number of program modules can be stored in secondary storage device 2014 and/or system memory 2004, including an operating system 2018, one or more application programs 2020, other program modules 2022 (such as the software engines described herein), and program data 2024. The computing device 2000 can utilize any suitable operating system, such as Microsoft Windows^{™}, Google Chrome^{™} OS, Apple OS, Unix, or Linux and variants and any other operating system suitable for a computing device. Other examples can include Microsoft, Google, or Apple operating systems, or any other suitable operating system used in tablet computing devices.

In some embodiments, a user provides inputs to the computing device 2000 through one or more input devices 2026. Examples of input devices 2026 include a keyboard 2028, mouse 2030, microphone 2032 (e.g., for voice and/or other audio input), touch sensor 2034 (such as a touchpad or touch sensitive display), and gesture sensor 2035 (e.g., for gestural input). In some implementations, the input device(s) 2026 provide detection based on presence, proximity, and/or motion. In some implementations, a user may walk into their home, and this may trigger an input into a processing device. For example, the input device(s) 2026 may then facilitate an automated experience for the user. Other embodiments include other input devices 2026. The input devices can be connected to the processing device 2002 through an input/output interface 2036 that is coupled to the system bus 2006. These input devices 2026 can be connected by any number of input/output interfaces, such as a parallel port, serial port, game port, or a universal serial bus. Wireless communication between input devices 2026 and the input/output interface 2036 is possible as well, and includes infrared, BLUETOOTH^{®} wireless technology, 802.11a/b/g/n, cellular, ultra-wideband (UWB), ZigBee, or other radio frequency communication systems in some possible embodiments, to name just a few examples.

In this example embodiment, a display device 2038, such as a monitor, liquid crystal display device, light-emitting diode display device, projector, or touch sensitive display device, is also connected to the system bus 2006 via an interface, such as a video adapter 2040. In addition to the display device 2038, the computing device 2000 can include various other peripheral devices (not shown), such as speakers or a printer.

The computing device 2000 can be connected to one or more networks through a network interface 2042. The network interface 2042 can provide for wired and/or wireless communication. In some implementations, the network interface 2042 can include one or more antennas for transmitting and/or receiving wireless signals. When used in a local area networking environment or a wide area networking environment (such as the Internet), the network interface 2042 can include an Ethernet interface. Other possible embodiments use other communication devices. For example, some embodiments of the computing device 2000 include a modem for communicating across the network.

The computing device 2000 can include at least some form of computer readable media. Computer readable media includes any available media that can be accessed by the computing device 2000. By way of example, computer readable media include computer readable storage media and computer readable communication media.

Computer readable storage media includes volatile and nonvolatile, removable and non-removable media implemented in any device configured to store information such as computer readable instructions, data structures, program modules or other data. Computer readable storage media includes, but is not limited to, random access memory, read only memory, electrically erasable programmable read only memory, flash memory or other memory technology, compact disc read only memory, digital versatile disks or other optical storage, magnetic cassettes, magnetic tape, magnetic disk storage or other magnetic storage devices, or any other medium that can be used to store the desired information and that can be accessed by the computing device 2000.

Computer readable communication media typically embodies computer readable instructions, data structures, program modules or other data in a modulated data signal such as a carrier wave or other transport mechanism and includes any information delivery media. The term "modulated data signal" refers to a signal that has one or more of its characteristics set or changed in such a manner as to encode information in the signal. By way of example, computer readable communication media includes wired media such as a wired network or direct-wired connection, and wireless media such as acoustic, radio frequency, infrared, and other wireless media. Combinations of any of the above are also included within the scope of computer readable media.

The computing device illustrated in FIG. 20 is also an example of programmable electronics, which may include one or more such computing devices, and when multiple computing devices are included, such computing devices can be coupled together with a suitable data communication network so as to collectively perform the various functions, methods, or operations disclosed herein.

The terms "substantially" and "about" used throughout this Specification are used to describe and account for small fluctuations, such as due to variations in processing. For example, they can refer to less than or equal to ±5%, such as less than or equal to ±2%, such as less than or equal to ±1%, such as less than or equal to ±0.5%, such as less than or equal to ±0.2%, such as less than or equal to 0.1%, such as less than or equal to 0.05%. Also, when used herein, an indefinite article such as "a" or "an" means "at least one."

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

A number of implementations have been described. Nevertheless, it will be understood that various modifications may be made without departing from the scope of the specification.

In addition, the logic flows depicted in the figures do not require the particular order shown, or sequential order, to achieve desirable results. In addition, other processes may be provided, or processes may be eliminated, from the described flows, and other components may be added to, or removed from, the described systems. Accordingly, other implementations are within the scope of the following claims.

## Claims

1. A computer-implemented method of detecting a missed bolus, the method comprising:
receiving blood glucose values and bolus information regarding a person with diabetes, the blood glucose values and the bolus information relating to a period of time;
generating, based on the blood glucose values and the bolus information, a meal probability density for the person, wherein meal probability density reflects a likelihood of meal ingestion with regard to time, and wherein generating the meal probability density comprises using a bolus density as a proxy for meal occurrence likelihood;
classifying, based on the blood glucose values and the bolus information, each of multiple time periods within the period of time regarding whether the time period is associated with ingestion of a meal by the person;
performing regression analysis on the classified multiple time periods to identify a first time period of the classified multiple time periods as corresponding to a beginning of the meal, wherein the bolus information indicates no bolus associated with the identified first time period;
associating, based on the regression analysis, a missed bolus event with the meal; and
issuing an alert regarding the person based on the missed bolus event being associated with the meal and taking into account the meal probability density before issuing the alert, wherein a relatively lower threshold for the alert is used when a current meal probability is relatively higher, wherein a relatively higher threshold for the alert is used when a current meal probability is relatively lower.

2. The computer-implemented method of claim 1, further comprising performing at least one action based on the missed bolus event being associated with the meal.

3. The computer-implemented method of claim 1, wherein the blood glucose values and the bolus information are received substantially in real time, and wherein the alert is issued substantially in real time with the bolus being missed;
optionally wherein issuing the alert in real time comprises waiting a predetermined time after the beginning of the meal before determining that the bolus was missed.

4. The computer-implemented method of any of claims 1 to 3, wherein classifying the multiple time periods comprises providing the blood glucose values and the bolus information to a first machine-learning set including at least one classifier, and wherein performing the regression analysis comprises providing at least an output of the first machine-learning set to a second machine-learning set including at least one regressor;
optionally wherein the first machine-learning set includes multiple classifiers receiving also the blood glucose values and the bolus information, and wherein classifying each of the multiple time periods comprises generating one classification event for each of the multiple classifiers for each of the multiple time periods,
and optionally further comprising providing each of the multiple classifiers with an aggregation of at least one of the blood glucose values or the bolus information.

5. The computer-implemented method of any of claims 1 to 4, wherein receiving the bolus information comprises receiving data generated by a pen cap based on the pen cap detecting at least one of a removal of the pen cap from an insulin pen, or a replacement of the pen cap on the insulin pen;
and/or wherein receiving the bolus information comprises receiving amount information corresponding to an amount of insulin administered to the person;
optionally further comprising:
following generating the meal probability density based on the blood glucose values and the bolus information, determining, based on the bolus information and the meal probability density, an expected bolus amount;
comparing the amount information with the expected bolus amount; and
issuing an alert regarding the person based on a discrepancy between the amount information and the expected bolus amount.

6. The computer-implemented method of claim 1, further comprising generating multiple meal probability densities corresponding to different days of a week for the person.

7. The computer-implemented method of claim 1, further comprising taking into account the meal probability density before associating the missed bolus event with the meal.

8. The computer-implemented method of claim 1, wherein taking into account the meal probability density comprises changing a threshold for a feature in a machine-learning classifier based on a kernel smoothed density estimate in the meal probability density;
and/or wherein taking into account the meal probability density comprises reducing a threshold for associating the missed bolus event with the meal.

9. The computer-implemented method of claim 1, further comprising generating the bolus density by aggregating bolus events for the person that are included in the bolus information.

10. The computer-implemented method of claim 9, wherein the bolus events are distributed within the period of time, and wherein aggregating the bolus events comprises:
wrapping the bolus events over a time interval shorter than the period of time so that the bolus events are distributed within the time interval;
generating, based on the bolus events wrapped over the time interval, a continuous distribution of likelihood over the time interval; and
replicating the continuous distribution of likelihood at least once within the period of time;
optionally wherein generating the continuous distribution of likelihood comprises smoothing the bolus events wrapped over the time interval;
and optionally wherein smoothing the bolus events comprises performing a kernel smoothed density estimate.

11. The computer-implemented method of claim 10, wherein generating the continuous distribution of likelihood comprises filtering the bolus events wrapped over the time interval.

12. The computer-implemented method of any of claims 1 to 11, further comprising generating, based on the blood glucose values and the bolus information, a bolus density for the person;
optionally wherein generating the bolus density comprises using a meal probability density for the person.

13. The computer-implemented method of any of claims 1 to 12, wherein the bolus information includes bolus events that are delta functions, the method further comprising broadening, before classifying the multiple time periods, each of the bolus events to have a finite time duration;
optionally wherein broadening each of the bolus events comprises convolving the delta function with a rectangle function;
and/or optionally wherein no bolus being associated with the identified first time period comprises that the bolus information includes no bolus separated from the identified first time period by at most a predefined time, and wherein the finite time duration is a multiple of the predefined time;
and/or optionally further comprising aggregating, before classifying the multiple time periods, the bolus events in multiple overlapping time intervals.

14. The computer-implemented method of any claims 1 to 13, wherein the blood glucose values and the bolus information are received substantially in real time;
and/or wherein the blood glucose values and the bolus information are received in batch;
and/or wherein no bolus being associated with the identified first time period comprises that the bolus information includes no bolus separated from the identified first time period by at most a predefined time, and wherein the bolus information does include a bolus separated from the identified first time period by more than the predefined time, the method further comprising associating a late bolus event with the bolus.

15. An insulin management system to determine a missed bolus comprising:
an input device to receive blood glucose values and bolus information regarding a person with diabetes, the blood glucose values and the bolus information relating to a period of time;
at least one processor in communication with the input device, wherein the processor is configured to:
generate, based on the blood glucose values and the bolus information, a meal probability density for the person, wherein meal probability density reflects a likelihood of meal ingestion with regard to time, and wherein generating the meal probability density comprises using a bolus density as a proxy for meal occurrence likelihood;
classify, based on the blood glucose values and the bolus information, each of multiple time periods within the period of time regarding whether the time period is associated with ingestion of a meal by the person; and
perform regression analysis on the classified multiple time periods to identify a first time period of the classified multiple time periods as corresponding to a beginning of the meal, wherein the bolus information indicates no bolus associated with the identified first time period;
associate, based on the regression analysis, a missed bolus event with the meal; and
issue an alert regarding the person based on the missed bolus event being associated with the meal and take into account the meal probability density before issuing the alert, wherein a relatively lower threshold for the alert is used when a current meal probability is relatively higher, wherein a relatively higher threshold for the alert is used when a current meal probability is relatively lower.

16. A computer program product stored in a non-transitory storage medium, the computer program product including instructions that when executed by at least one processor cause the at least one processor to perform operations comprising:
receiving blood glucose values and bolus information regarding a person with diabetes, the blood glucose values and the bolus information relating to a period of time;
generating, based on the blood glucose values and the bolus information, a meal probability density for the person, wherein meal probability density reflects a likelihood of meal ingestion with regard to time, and wherein generating the meal probability density comprises using a bolus density as a proxy for meal occurrence likelihood;
classifying, based on the blood glucose values and the bolus information, each of multiple time periods within the period of time regarding whether the time period is associated with ingestion of a meal by the person;
performing regression analysis on the classified multiple time periods to identify a first time period of the classified multiple time periods as corresponding to a beginning of the meal, wherein the bolus information indicates no bolus associated with the identified first time period; and
associating, based on the regression analysis, a missed bolus event with the meal; and
issuing an alert regarding the person based on the missed bolus event being associated with the meal and taking into account the meal probability density before issuing the alert, wherein a relatively lower threshold for the alert is used when a current meal probability is relatively higher, wherein a relatively higher threshold for the alert is used when a current meal probability is relatively lower.

## Patentansprüche

1. Ein computerimplementiertes Verfahren zum Detektieren eines verpassten Bolus, wobei das Verfahren Folgendes beinhaltet:
Empfangen von Blutzuckerwerten und Bolusinformationen bezüglich einer Person mit Diabetes, wobei sich die Blutzuckerwerte und die Bolusinformationen auf einen Zeitraum beziehen;
Erzeugen, basierend auf den Blutzuckerwerten und den Bolusinformationen, einer Mahlzeitwahrscheinlichkeitsdichte für die Person, wobei die
Mahlzeitwahrscheinlichkeitsdichte eine Wahrscheinlichkeit einer Mahlzeitaufnahme bezüglich der Zeit widerspiegelt und wobei das Erzeugen der
Mahlzeitwahrscheinlichkeitsdichte das Verwenden einer Bolusdichte als einen Proxy für die Wahrscheinlichkeit des Auftretens einer Mahlzeit beinhaltet;
Klassifizieren, basierend auf den Blutzuckerwerten und den Bolusinformationen, jedes von mehreren Zeiträumen innerhalb des Zeitraums bezüglich dessen, ob der Zeitraum mit der Aufnahme einer Mahlzeit durch die Person assoziiert ist;
Durchführen einer Regressionsanalyse an den klassifizierten mehreren Zeiträumen, um einen ersten Zeitraum der klassifizierten mehreren Zeiträume als einem Beginn der Mahlzeit entsprechend zu identifizieren, wobei die Bolusinformationen keinen Bolus angeben, der mit dem identifizierten ersten Zeitraum assoziiert ist;
Assoziieren, basierend auf der Regressionsanalyse, eines verpassten Bolusereignisses mit der Mahlzeit; und
Ausgeben einer Warnung bezüglich der Person basierend darauf, dass das verpasste Bolusereignis mit der Mahlzeit assoziiert ist, und Berücksichtigen der Mahlzeitwahrscheinlichkeitsdichte vor dem Ausgeben der Warnung, wobei ein relativ niedrigerer Schwellenwert für die Warnung verwendet wird, wenn eine aktuelle Mahlzeitwahrscheinlichkeit relativ höher ist, wobei ein relativ höherer Schwellenwert für die Warnung verwendet wird, wenn eine aktuelle Mahlzeitwahrscheinlichkeit relativ niedriger ist.

2. Computerimplementiertes Verfahren gemäß Anspruch 1, das ferner das Durchführen mindestens einer Handlung basierend darauf, dass das verpasste Bolusereignis mit der Mahlzeit assoziiert ist, beinhaltet.

3. Computerimplementiertes Verfahren gemäß Anspruch 1, wobei die Blutzuckerwerte und die Bolusinformationen im Wesentlichen in Echtzeit empfangen werden und wobei die Warnung im Wesentlichen in Echtzeit ausgegeben wird, wenn der Bolus verpasst ist;
wobei optional das Ausgeben der Warnung in Echtzeit das Warten eine vorbestimmte Zeit nach dem Beginn der Mahlzeit beinhaltet, bevor bestimmt wird, dass der Bolus verpasst wurde.

4. Computerimplementiertes Verfahren gemäß einem der Ansprüche 1 bis 3, wobei das Klassifizieren der mehreren Zeiträume das Bereitstellen der Blutzuckerwerte und der Bolusinformationen an einen ersten mindestens einen Klassifikator umfassenden Maschinenlernsatz beinhaltet und wobei das Durchführen der Regressionsanalyse das Bereitstellen mindestens einer Ausgabe des ersten Maschinenlernsatzes an einen zweiten mindestens einen Regressor umfassenden Maschinenlernsatz beinhaltet; wobei optional der erste Maschinenlernsatz mehrere Klassifikatoren umfasst, die auch die Blutzuckerwerte und die Bolusinformationen empfangen, und wobei das Klassifizieren jedes der mehreren Zeiträume das Erzeugen eines Klassifizierungsereignisses für jeden der mehreren Klassifikatoren für jeden der mehreren Zeiträume beinhaltet,
und optional ferner beinhaltend das Versehen jedes der mehreren Klassifikatoren mit einer Aggregation von mindestens einem von den Blutzuckerwerten oder den Bolusinformationen.

5. Computerimplementiertes Verfahren gemäß einem der Ansprüche 1 bis 4, wobei das Empfangen der Bolusinformationen das Empfangen von Daten beinhaltet, die von einer Stiftkappe basierend darauf erzeugt werden, dass die Stiftkappe mindestens eines von einem Entfernen der Stiftkappe von einem Insulinstift oder einem Ersetzen der Stiftkappe auf dem Insulinstift detektiert;
und/oder wobei das Empfangen der Bolusinformationen das Empfangen von Mengeninformationen beinhaltet, die einer der Person verabreichten Insulinmenge entsprechen;
optional ferner beinhaltend:
nach dem Erzeugen der Mahlzeitwahrscheinlichkeitsdichte basierend auf den Blutzuckerwerten und den Bolusinformationen, Bestimmen, basierend auf den Bolusinformationen und der Mahlzeitwahrscheinlichkeitsdichte, einer erwarteten Bolusmenge;
Vergleichen der Mengeninformationen mit der erwarteten Bolusmenge; und
Ausgeben einer Warnung bezüglich der Person basierend auf einer Diskrepanz zwischen den Mengeninformationen und der erwarteten Bolusmenge.

6. Computerimplementiertes Verfahren gemäß Anspruch 1, das ferner das Erzeugen mehrerer Mahlzeitwahrscheinlichkeitsdichten beinhaltet, die verschiedenen Tagen einer Woche für die Person entsprechen.

7. Computerimplementiertes Verfahren gemäß Anspruch 1, das ferner das Berücksichtigen der Mahlzeitwahrscheinlichkeitsdichte vor dem Assoziieren des verpassten Bolusereignisses mit der Mahlzeit beinhaltet.

8. Computerimplementiertes Verfahren gemäß Anspruch 1, wobei das Berücksichtigen der Mahlzeitwahrscheinlichkeitsdichte das Ändern eines Schwellenwerts für ein Merkmal in einem Maschinenlernklassifikator basierend auf einer kernelgeglätteten Dichteschätzung in der Mahlzeitwahrscheinlichkeitsdichte beinhaltet;
und/oder wobei das Berücksichtigen der Mahlzeitwahrscheinlichkeitsdichte das Reduzieren eines Schwellenwerts zum Assoziieren des verpassten Bolusereignisses mit der Mahlzeit beinhaltet.

9. Computerimplementiertes Verfahren gemäß Anspruch 1, das ferner das Erzeugen der Bolusdichte durch Aggregieren von Bolusereignissen für die Person, die in den Bolusinformationen umfasst sind, beinhaltet.

10. Computerimplementiertes Verfahren gemäß Anspruch 9, wobei die Bolusereignisse innerhalb des Zeitraums verteilt sind und wobei das Aggregieren der Bolusereignisse Folgendes beinhaltet:
Umhüllen der Bolusereignisse über ein Zeitintervall, das kürzer als der Zeitraum ist, sodass die Bolusereignisse innerhalb des Zeitintervalls verteilt sind;
Erzeugen, basierend auf den über das Zeitintervall umhüllten Bolusereignissen, einer kontinuierlichen Wahrscheinlichkeitsverteilung über das Zeitintervall; und
Replizieren der kontinuierlichen Wahrscheinlichkeitsverteilung mindestens einmal innerhalb des Zeitraums;
wobei optional das Erzeugen der kontinuierlichen Wahrscheinlichkeitsverteilung das Glätten der über das Zeitintervall umhüllten Bolusereignisse beinhaltet;
und wobei optional das Glätten der Bolusereignisse das Durchführen einer kernelgeglätteten Dichteschätzung beinhaltet.

11. Computerimplementiertes Verfahren gemäß Anspruch 10, wobei das Erzeugen der kontinuierlichen Wahrscheinlichkeitsverteilung das Filtern der über das Zeitintervall umhüllten Bolusereignisse beinhaltet.

12. Computerimplementiertes Verfahren gemäß einem der Ansprüche 1 bis 11, das ferner das Erzeugen, basierend auf den Blutzuckerwerten und den Bolusinformationen, einer Bolusdichte für die Person beinhaltet;
wobei optional das Erzeugen der Bolusdichte das Verwenden einer Mahlzeitwahrscheinlichkeitsdichte für die Person beinhaltet.

13. Computerimplementiertes Verfahren gemäß einem der Ansprüche 1 bis 12, wobei die Bolusinformationen Bolusereignisse umfassen, die Deltafunktionen sind, wobei das Verfahren ferner das Erweitern, vor dem Klassifizieren der mehreren Zeiträume, jedes der Bolusereignisse, sodass es eine endliche Zeitdauer aufweist, beinhaltet;
wobei optional das Erweitern jedes der Bolusereignisse das Falten der Deltafunktion mit einer Rechteckfunktion beinhaltet;
und/oder wobei optional, dass kein Bolus mit dem identifizierten ersten Zeitraum assoziiert ist, beinhaltet, dass die Bolusinformationen keinen Bolus umfassen, der von dem identifizierten ersten Zeitraum um höchstens eine vordefinierte Zeit getrennt ist, und wobei die endliche Zeitdauer ein Vielfaches der vordefinierten Zeit ist;
und/oder optional ferner beinhaltend das Aggregieren, vor dem Klassifizieren der mehreren Zeiträume, der Bolusereignisse in mehreren überlappenden Zeitintervallen.

14. Computerimplementiertes Verfahren gemäß einem der Ansprüche 1 bis 13, wobei die Blutzuckerwerte und die Bolusinformationen im Wesentlichen in Echtzeit empfangen werden;
und/oder wobei die Blutzuckerwerte und die Bolusinformationen chargenweise empfangen werden;
und/oder wobei, dass kein Bolus mit dem identifizierten ersten Zeitraum assoziiert ist, beinhaltet, dass die Bolusinformationen keinen Bolus umfassen, der von dem identifizierten ersten Zeitraum um höchstens eine vordefinierte Zeit getrennt ist, und
wobei die Bolusinformationen einen Bolus umfassen, der von dem identifizierten ersten Zeitraum um mehr als die vordefinierte Zeit getrennt ist, wobei das Verfahren ferner das Assoziieren eines späten Bolusereignisses mit dem Bolus beinhaltet.

15. Ein Insulinmanagementsystem zum Bestimmen eines verpassten Bolus, das Folgendes beinhaltet:
eine Eingabevorrichtung zum Empfangen von Blutzuckerwerten und Bolusinformationen bezüglich einer Person mit Diabetes, wobei sich die Blutzuckerwerte und die Bolusinformationen auf einen Zeitraum beziehen;
mindestens einen Prozessor in Kommunikation mit der Eingabevorrichtung, wobei der Prozessor für Folgendes konfiguriert ist:
Erzeugen, basierend auf den Blutzuckerwerten und den Bolusinformationen, einer Mahlzeitwahrscheinlichkeitsdichte für die Person, wobei die Mahlzeitwahrscheinlichkeitsdichte eine Wahrscheinlichkeit einer Mahlzeitaufnahme bezüglich der Zeit widerspiegelt und wobei das Erzeugen der Mahlzeitwahrscheinlichkeitsdichte das Verwenden einer Bolusdichte als einen Proxy für die Wahrscheinlichkeit des Auftretens einer Mahlzeit beinhaltet;
Klassifizieren, basierend auf den Blutzuckerwerten und den Bolusinformationen, jedes von mehreren Zeiträumen innerhalb des Zeitraums bezüglich dessen, ob der Zeitraum mit der Aufnahme einer Mahlzeit durch die Person assoziiert ist; und
Durchführen einer Regressionsanalyse an den klassifizierten mehreren Zeiträumen, um einen ersten Zeitraum der klassifizierten mehreren Zeiträume als einem Beginn der Mahlzeit entsprechend zu identifizieren, wobei die Bolusinformationen keinen Bolus angeben, der mit dem identifizierten ersten Zeitraum assoziiert ist;
Assoziieren, basierend auf der Regressionsanalyse, eines verpassten Bolusereignisses mit der Mahlzeit; und
Ausgeben einer Warnung bezüglich der Person basierend darauf, dass das verpasste Bolusereignis mit der Mahlzeit assoziiert ist, und Berücksichtigen der Mahlzeitwahrscheinlichkeitsdichte vor dem Ausgeben der Warnung, wobei ein relativ niedrigerer Schwellenwert für die Warnung verwendet wird, wenn eine aktuelle Mahlzeitwahrscheinlichkeit relativ höher ist, wobei ein relativ höherer Schwellenwert für die Warnung verwendet wird, wenn eine aktuelle Mahlzeitwahrscheinlichkeit relativ niedriger ist.

16. Ein Computerprogrammprodukt, das in einem nichttransitorischen Speichermedium gespeichert ist, wobei das Computerprogrammprodukt Anweisungen umfasst, die, wenn sie von mindestens einem Prozessor ausgeführt werden, den mindestens einen Prozessor veranlassen, Operationen durchzuführen, die Folgendes beinhalten:
Empfangen von Blutzuckerwerten und Bolusinformationen bezüglich einer Person mit Diabetes, wobei sich die Blutzuckerwerte und die Bolusinformationen auf einen Zeitraum beziehen;
Erzeugen, basierend auf den Blutzuckerwerten und den Bolusinformationen, einer Mahlzeitwahrscheinlichkeitsdichte für die Person, wobei die Mahlzeitwahrscheinlichkeitsdichte eine Wahrscheinlichkeit einer Mahlzeitaufnahme bezüglich der Zeit widerspiegelt und wobei das Erzeugen der Mahlzeitwahrscheinlichkeitsdichte das Verwenden einer Bolusdichte als einen Proxy für die Wahrscheinlichkeit des Auftretens einer Mahlzeit beinhaltet;
Klassifizieren, basierend auf den Blutzuckerwerten und den Bolusinformationen, jedes von mehreren Zeiträumen innerhalb des Zeitraums bezüglich dessen, ob der Zeitraum mit der Aufnahme einer Mahlzeit durch die Person assoziiert ist;
Durchführen einer Regressionsanalyse an den klassifizierten mehreren Zeiträumen, um einen ersten Zeitraum der klassifizierten mehreren Zeiträume als einem Beginn der Mahlzeit entsprechend zu identifizieren, wobei die Bolusinformationen keinen Bolus angeben, der mit dem identifizierten ersten Zeitraum assoziiert ist; und
Assoziieren, basierend auf der Regressionsanalyse, eines verpassten Bolusereignisses mit der Mahlzeit; und
Ausgeben einer Warnung bezüglich der Person basierend darauf, dass das verpasste Bolusereignis mit der Mahlzeit assoziiert ist, und Berücksichtigen der Mahlzeitwahrscheinlichkeitsdichte vor dem Ausgeben der Warnung, wobei ein relativ niedrigerer Schwellenwert für die Warnung verwendet wird, wenn eine aktuelle Mahlzeitwahrscheinlichkeit relativ höher ist, wobei ein relativ höherer Schwellenwert für die Warnung verwendet wird, wenn eine aktuelle Mahlzeitwahrscheinlichkeit relativ niedriger ist.

## Revendications

1. Un procédé mis en œuvre par ordinateur de détection d'un bolus manqué, le procédé comprenant :
le fait de recevoir des valeurs de glycémie et des informations de bolus concernant une personne atteinte de diabète, les valeurs de glycémie et les informations de bolus se rapportant à une période de temps ;
le fait de générer, sur la base des valeurs de glycémie et des informations de bolus,
une densité de probabilité de repas pour la personne, la densité de probabilité de repas reflétant une vraisemblance d'ingestion de repas relativement au temps, et le fait de générer la densité de probabilité de repas comprenant le fait d'utiliser une densité de bolus comme substitut pour la vraisemblance d'occurrence de repas ;
le fait de classer, sur la base des valeurs de glycémie et des informations de bolus,
chacune de multiples périodes de temps au sein de la période de temps quant au fait que la période de temps est associée ou non à l'ingestion d'un repas par la personne ;
le fait d'effectuer une analyse de régression sur les multiples périodes de temps classées pour identifier une première période de temps des multiples périodes de temps classées comme correspondant à un début du repas, les informations de bolus indiquant qu'aucun bolus n'est associé à la première période de temps identifiée ;
le fait d'associer, sur la base de l'analyse de régression, un événement de bolus manqué au repas ; et
le fait d'émettre une alerte concernant la personne sur la base du fait que l'événement de bolus manqué est associé au repas et de prendre en compte la densité de probabilité de repas avant d'émettre l'alerte, un seuil relativement plus bas pour l'alerte étant utilisé lorsqu'une probabilité de repas actuelle est relativement plus élevée, un seuil relativement plus élevé pour l'alerte étant utilisé lorsqu'une probabilité de repas actuelle est relativement plus basse.

2. Le procédé mis en œuvre par ordinateur de la revendication 1, comprenant en outre le fait d'effectuer au moins une action sur la base du fait que l'événement de bolus manqué est associé au repas.

3. Le procédé mis en œuvre par ordinateur de la revendication 1, dans lequel les valeurs de glycémie et les informations de bolus sont reçues substantiellement en temps réel, et dans lequel l'alerte est émise substantiellement en temps réel avec le fait que le bolus soit manqué ;
facultativement dans lequel le fait d'émettre l'alerte en temps réel comprend le fait d'attendre un temps prédéterminé après le début du repas avant de déterminer que le bolus a été manqué.

4. Le procédé mis en œuvre par ordinateur de n'importe lesquelles des revendications 1 à 3, dans lequel le fait de classer les multiples périodes de temps comprend le fait de fournir les valeurs de glycémie et les informations de bolus à un premier ensemble d'apprentissage machine incluant au moins un classificateur, et dans lequel le fait d'effectuer l'analyse de régression comprend le fait de fournir au moins une sortie du premier ensemble d'apprentissage machine à un deuxième ensemble d'apprentissage machine incluant au moins un régresseur ;
facultativement dans lequel le premier ensemble d'apprentissage machine inclut de multiples classificateurs recevant également les valeurs de glycémie et les informations de bolus, et dans lequel le fait de classer chacune des multiples périodes de temps comprend le fait de générer un événement de classification pour chacun des multiples classificateurs pour chacune des multiples périodes de temps,
et facultativement comprenant en outre le fait de fournir à chacun des multiples classificateurs une agrégation d'au moins un élément parmi les valeurs de glycémie ou les informations de bolus.

5. Le procédé mis en œuvre par ordinateur de n'importe lesquelles des revendications 1 à 4, dans lequel le fait de recevoir les informations de bolus comprend le fait de recevoir des données générées par un capuchon de stylo sur la base du fait que le capuchon de stylo détecte au moins un élément parmi un retrait du capuchon de stylo d'un stylo à insuline, ou un replacement du capuchon de stylo sur le stylo à insuline ;
et/ou dans lequel le fait de recevoir les informations de bolus comprend le fait de recevoir des informations de quantité correspondant à une quantité d'insuline administrée à la personne ;
facultativement comprenant en outre :
suite à la génération de la densité de probabilité de repas sur la base des valeurs de glycémie et des informations de bolus, le fait de déterminer, sur la base des informations de bolus et de la densité de probabilité de repas, une quantité de bolus attendue ;
le fait de comparer les informations de quantité à la quantité de bolus attendue ; et
le fait d'émettre une alerte concernant la personne sur la base d'une divergence entre les informations de quantité et la quantité de bolus attendue.

6. Le procédé mis en œuvre par ordinateur de la revendication 1, comprenant en outre le fait de générer de multiples densités de probabilité de repas correspondant à différents jours d'une semaine pour la personne.

7. Le procédé mis en œuvre par ordinateur de la revendication 1, comprenant en outre le fait de prendre en compte la densité de probabilité de repas avant d'associer l'événement de bolus manqué au repas.

8. Le procédé mis en œuvre par ordinateur de la revendication 1, dans lequel le fait de prendre en compte la densité de probabilité de repas comprend le fait de changer un seuil pour une caractéristique dans un classificateur d'apprentissage machine sur la base d'une estimation de densité lissée par noyau dans la densité de probabilité de repas ;
et/ou dans lequel le fait de prendre en compte la densité de probabilité de repas comprend le fait de réduire un seuil pour associer l'événement de bolus manqué au repas.

9. Le procédé mis en œuvre par ordinateur de la revendication 1, comprenant en outre le fait de générer la densité de bolus en agrégeant des événements de bolus pour la personne qui sont inclus dans les informations de bolus.

10. Le procédé mis en œuvre par ordinateur de la revendication 9, dans lequel les événements de bolus sont distribués au sein de la période de temps, et dans lequel le fait d'agréger les événements de bolus comprend :
le fait d'enrouler les événements de bolus sur un intervalle de temps plus court que la période de temps de sorte que les événements de bolus soient distribués au sein de l'intervalle de temps ;
le fait de générer, sur la base des événements de bolus enroulés sur l'intervalle de temps, une distribution continue de vraisemblance sur l'intervalle de temps ; et
le fait de répliquer la distribution continue de vraisemblance au moins une fois au sein de la période de temps ;
facultativement dans lequel le fait de générer la distribution continue de vraisemblance comprend le fait de lisser les événements de bolus enroulés sur l'intervalle de temps ;
et facultativement dans lequel le fait de lisser les événements de bolus comprend le fait d'effectuer une estimation de densité lissée par noyau.

11. Le procédé mis en œuvre par ordinateur de la revendication 10, dans lequel le fait de générer la distribution continue de vraisemblance comprend le fait de filtrer les événements de bolus enroulés sur l'intervalle de temps.

12. Le procédé mis en œuvre par ordinateur de n'importe lesquelles des revendications 1 à 11, comprenant en outre le fait de générer, sur la base des valeurs de glycémie et des informations de bolus, une densité de bolus pour la personne ;
facultativement dans lequel le fait de générer la densité de bolus comprend le fait d'utiliser une densité de probabilité de repas pour la personne.

13. Le procédé mis en œuvre par ordinateur de n'importe lesquelles des revendications 1 à 12, dans lequel les informations de bolus incluent des événements de bolus qui sont des fonctions delta, le procédé comprenant en outre le fait d'élargir, avant de classer les multiples périodes de temps, chacun des événements de bolus pour avoir une durée de temps finie ;
facultativement dans lequel le fait d'élargir chacun des événements de bolus comprend le fait de convoluer la fonction delta avec une fonction rectangle ;
et/ou facultativement dans lequel le fait qu'aucun bolus n'est associé à la première période de temps identifiée comprend le fait que les informations de bolus n'incluent aucun bolus séparé de la première période de temps identifiée d'au plus un temps prédéfini, et dans lequel la durée de temps finie est un multiple du temps prédéfini ; et/ou facultativement comprenant en outre le fait d'agréger, avant de classer les multiples périodes de temps, les événements de bolus dans de multiples intervalles de temps se chevauchant.

14. Le procédé mis en œuvre par ordinateur de n'importe quelles revendications 1 à 13, dans lequel les valeurs de glycémie et les informations de bolus sont reçues substantiellement en temps réel ;
et/ou dans lequel les valeurs de glycémie et les informations de bolus sont reçues par lots ;
et/ou dans lequel le fait qu'aucun bolus n'est associé à la première période de temps identifiée comprend le fait que les informations de bolus n'incluent aucun bolus séparé de la première période de temps identifiée d'au plus un temps prédéfini, et dans lequel les informations de bolus incluent un bolus séparé de la première période de temps identifiée de plus du temps prédéfini, le procédé comprenant en outre le fait d'associer un événement de bolus tardif au bolus.

15. Un système de gestion d'insuline pour déterminer un bolus manqué comprenant :
un dispositif d'entrée pour recevoir des valeurs de glycémie et des informations de bolus concernant une personne atteinte de diabète, les valeurs de glycémie et les informations de bolus se rapportant à une période de temps ;
au moins un processeur en communication avec le dispositif d'entrée, le processeur étant configuré pour :
générer, sur la base des valeurs de glycémie et des informations de bolus, une densité de probabilité de repas pour la personne, la densité de probabilité de repas reflétant une vraisemblance d'ingestion de repas relativement au temps, et le fait de générer la densité de probabilité de repas comprenant le fait d'utiliser une densité de bolus comme substitut pour la vraisemblance d'occurrence de repas ;
classer, sur la base des valeurs de glycémie et des informations de bolus, chacune de multiples périodes de temps au sein de la période de temps quant au fait que la période de temps est associée ou non à l'ingestion d'un repas par la personne ; et
effectuer une analyse de régression sur les multiples périodes de temps classées pour identifier une première période de temps des multiples périodes de temps classées comme correspondant à un début du repas, les informations de bolus indiquant qu'aucun bolus n'est associé à la première période de temps identifiée ;
associer, sur la base de l'analyse de régression, un événement de bolus manqué au repas ; et
émettre une alerte concernant la personne sur la base du fait que l'événement de bolus manqué est associé au repas et prendre en compte la densité de probabilité de repas avant d'émettre l'alerte, un seuil relativement plus bas pour l'alerte étant utilisé lorsqu'une probabilité de repas actuelle est relativement plus élevée, un seuil relativement plus élevé pour l'alerte étant utilisé lorsqu'une probabilité de repas actuelle est relativement plus basse.

16. Un produit de programme informatique stocké dans un support de stockage non transitoire, le produit de programme informatique incluant des instructions qui, lorsqu'elles sont exécutées par au moins un processeur, amènent l'au moins un processeur à effectuer des opérations comprenant :
le fait de recevoir des valeurs de glycémie et des informations de bolus concernant une personne atteinte de diabète, les valeurs de glycémie et les informations de bolus se rapportant à une période de temps ;
le fait de générer, sur la base des valeurs de glycémie et des informations de bolus,
une densité de probabilité de repas pour la personne, la densité de probabilité de repas reflétant une vraisemblance d'ingestion de repas relativement au temps, et le fait de générer la densité de probabilité de repas comprenant le fait d'utiliser une densité de bolus comme substitut pour la vraisemblance d'occurrence de repas ;
le fait de classer, sur la base des valeurs de glycémie et des informations de bolus,
chacune de multiples périodes de temps au sein de la période de temps quant au fait que la période de temps est associée ou non à l'ingestion d'un repas par la personne ;
le fait d'effectuer une analyse de régression sur les multiples périodes de temps classées pour identifier une première période de temps des multiples périodes de temps classées comme correspondant à un début du repas, les informations de bolus indiquant qu'aucun bolus n'est associé à la première période de temps identifiée ; et
le fait d'associer, sur la base de l'analyse de régression, un événement de bolus manqué au repas ; et
le fait d'émettre une alerte concernant la personne sur la base du fait que l'événement de bolus manqué est associé au repas et de prendre en compte la densité de probabilité de repas avant d'émettre l'alerte, un seuil relativement plus bas pour l'alerte étant utilisé lorsqu'une probabilité de repas actuelle est relativement plus élevée, un seuil relativement plus élevé pour l'alerte étant utilisé lorsqu'une probabilité de repas actuelle est relativement plus basse.
